# EUROPEAN PATENT SPECIFICATION

(11) **EP 3 986 566 B1**
(45) Date of publication and mention of the grant of the patent: **30.08.2023**
(21) Application number: 20734487.0
(22) Date of filing: 19.06.2020
(51) Int. Cl.: A61P 35/00, C07D 417/14, A61K 31/427

(54) **NEW EGFR INHIBITORS**
NEUE EGFR-INHIBITOREN
NOUVEAUX INHIBITEURS DE L'EGFR

(30) Priority: 21.06.2019 EP 19181772
(43) Date of publication of application: 27.04.2022
(73) Proprietor: F. Hoffmann-La Roche AG, 4070 Basel (CH)
(72) Inventor: DOLENTE, Cosimo, 4070 BASEL (CH); GOERGLER, Annick, 4070 Basel (CH); HEWINGS, David Stephen, 4070 Basel (CH); JAESCHKE, Georg, 4070 Basel (CH); KUHN, Bernd, 4070 Basel (CH); NAGEL, Yvonne Alice, 4070 Basel (CH); NORCROSS, Roger David, 4070 Basel (CH); OBST-SANDER, Christa Ulrike, 4070 Basel (CH); RICCI, Antonio, 4070 Basel (CH); RUEHER, Daniel, 4070 Basel (CH); STEINER, Sandra, 4070 Basel (CH)
(74) Representative: Salud, Carlos E.
(86) International application number: PCT/EP2020/067055
(87) International publication number: WO 2020/254546

(56) References cited:
- WO-A1-2018/220149
- US-A1- 2018 290 975

## Description

The present invention provides compounds which are selective allosteric inhibitors of T790M/L858R, T790M/L858R/C797S, L858R, L858R/C797S containing EGFR mutants, their manufacture, pharmaceutical compositions containing them and their use as therapeutically active substances.

The present invention provides a novel compounds selected from
2-(6,7-dihydro-5H-pyrrolo[1,2-c]imidazol-1-yl)-2-[6-[4-(1-ethyl-4-piperidyl)phenyl]-1-oxo-4-(trifluoromethyl)isoindolin-2-yl]-N-thiazol-2-yl-acetamide;
2-(6,7-dihydro-5H-pyrrolo[1,2-c]imidazol-1-yl)-2-[6-[2-[4-[[4-(hydroxymethyl)-1-piperidyl]methyl]phenyl]ethynyl]-1-oxo-4-(trifluoromethyl)isoindolin-2-yl]-N-thiazol-2-yl-acetamide;
2-[4-(difluoromethyl)-6-[4-(1-ethyl-4-piperidyl)phenyl]-1-oxo-isoindolin-2-yl]-2-(6,7-dihydro-5H-pyrrolo[1,2-c]imidazol-1-yl)-N-thiazol-2-yl-acetamide;
2-[4-(difluoromethyl)-6-[2-[4-[[4-(hydroxymethyl)-1-piperidyl]methyl]phenyl]ethynyl]-1-oxo-isoindolin-2-yl]-2-(6,7-dihydro-5H-pyrrolo[1,2-c]imidazol-1-yl)-N-thiazol-2-yl-acetamide;
2-[6- [2-[4-[[4-(hydroxymethyl)-1-piperidyl] methyl]phenyl]ethynyl] -1 -oxo-4-(trifluoromethyl)isoindolin-2-yl]-2-[rac-(6R)-6-fluoro-6,7-dihydro-5H-pyrrolo[1,2-c]imidazol-1-yl]-N-thiazol-2-yl-acetamide;
2-[4-(difluoromethyl)-6-[4-(1-ethyl-4-piperidyl)phenyl]-1-oxo-isoindolin-2-yl]-2-[rac-(6R)-6-fluoro-6,7-dihydro-5H-pyrrolo[1,2-c]imidazol-1-yl]-N-thiazol-2-yl-acetamide;
2-[4-(difluoromethyl)-1-oxo-6-[4-[rac-(3R,4R)-1-ethyl-3-fluoro-4-piperidyl]phenyl]isoindolin-2-yl]-2-(6,7-dihydro-5H-pyrrolo[1,2-c]imidazol-1-yl)-N-thiazol-2-yl-acetamide;
2-[6-[2-[4-[(6-hydroxy-2-azaspiro[3.3]heptan-2-yl)methyl]phenyl]ethynyl]-1-oxo-4-(trifluoromethyl)isoindolin-2-yl]-2-[rac-(6R)-6-fluoro-6,7-dihydro-5H-pyrrolo[1,2-c]imidazol-1-yl]-N-thiazol-2-yl-acetamide;
2-[4-(difluoromethyl)-6-[2-[4-[[4-(hydroxymethyl)-1-piperidyl]methyl]phenyl]ethynyl]-1-oxo-isoindolin-2-yl]-2-[rac-(6R)-6-fluoro-6,7-dihydro-5H-pyrrolo[1,2-c]imidazol-1-yl]-N-thiazol-2-yl-acetamide;
2-[6-[2-[4-[(4-hydroxyazepan-1-yl)methyl]phenyl] ethynyl]-1-oxo-4-(trifluoromethyl)isoindolin-2-yl]-2-[rac-(6R)-6-fluoro-6,7-dihydro-5H-pyrrolo[1,2-c]imidazol-1-yl]-N-thiazol-2-yl-acetamide;
or pharmaceutically acceptable salts.

The HER family receptor tyrosine kinases are mediators of cell growth, differentiation and survival. The receptor family includes four distinct members, i.e. epidermal growth factor receptor (EGFR, ErbBl, or HER1) HER2 (ErbB2), HER3 (ErbB3) and HER4 (ErbB4). Upon ligand binding the receptors form homo and heterodimers and subsequent activation of the intrinsic tyrosine kinase activity leads to receptor auto-phosphorylation and the activation of downstream signaling molecules (Yarden, Y., Sliwkowski, MX. Untangling the ErbB signalling network. Nature Review Mol Cell Biol. 2001 Feb;2(2): 127-37). De-regulation of EGFR by overexpression or mutation has been implicated in many types of human cancer including colorectal, pancreatic, gliomas, head and neck and lung cancer, in particular non-small cell lung cancer (NSCLC) and several EGFR targeting agents have been developed over the years (Ciardiello, F., and Tortora, G. (2008). EGFR antagonists in cancer treatment. The New England journal of medicine 358, 1160-1174). Erlotinib (Tarceva^{®}), a reversible inhibitor of the EGFR tyrosine kinase was approved in numerous countries for the treatment of recurrent NSCLC.

An impressive single agent activity of EGFR tyrosine kinase inhibitors is observed in a subset ofNSCLC patients whose tumors harbor somatic kinase domain mutations, whereas clinical benefit in wild-type EGFR patients is greatly diminished (Paez, J. et al. (2004). EGFR mutations in lung cancer: correlation with clinical response to gefitinib therapy. Science (New York, NY 304, 1497-1500). The most common somatic mutations of EGFR are exon 19 deletions with delta 746-750 the most prevalent mutation and the exon 21 amino acid substitutions with L858R the most frequent mutation (Sharma SV, Bell DW, Settleman J, Haber DA. Epidermal growth factor receptor mutations in lung cancer. Nat Rev Cancer. 2007 Mar;7(3): 169-81).

Treatment resistance arises frequently, often due to the secondary T790M mutation within the ATP site of the receptor. Some developed mutant-selective irreversible inhibitors are highly active against the T790M mutant, but their efficacy can be compromised by acquired mutation of C797S, that is the cysteine residue with which they form a key covalent bond (Thress, K. S. et al. Acquired EGFR C797S mutation mediates resistance to AZD9291 in non-small cell lung cancer harboring EGFR T790M. Nat. Med. 21, 560-562 (2015)). C797S mutation was further reported by Wang to be a major mechanism for resistance to T790M-targeting EGFR inhibitors (Wang et al. EGFR C797S mutation mediates resistance to third-generation inhibitors in T790M-positive non-small cell lung cancer, J Hematol Oncol. 2016; 9: 59). Additional mutations that cause resistance to Osimertinib are described by Yang, for example L718Q.( Yang et al, Investigating Novel Resistance Mechanisms to Third-Generation EGFR Tyrosine Kinase Inhibitor Osimertinib in Non-Small Cell Lung Cancer Patients, Clinical Cancer Research, DOI: 10.1158/1078-0432.CCR-17-2310) Lu et al.( Targeting EGFRL858R/T790M and EGFRL858R/T790M/C797S resistance mutations in NSCLC: Current developments in medicinal chemistry, Med Res Rev 2018; 1-32) report in a review article on Targeting EGFR^{L858R/T790M} and EGFR^{L858R/T790M/C797S} resistance mutations in NSCLC treatment.

As most available EGFR tyrosine kinase inhibitors target the ATP-site of the kinase, there is a need for new therapeutic agents that work differently, for example through targeting drug-resistant EGFR mutants.

Recent studies suggest that purposefully targeting allosteric sites might lead to mutant-selective inhibitors (Jia et al. Overcoming EGFR(T790M) and EGFR(C797S) resistance with mutant-selective allosteric inhibitoRS, June 2016, Nature 534, 129-132)

There is just a need in the generation of selective molecules that specifically inhibit T790M/L858R, T790M/L858R/C797S, L858R, L858R/C797S containing EGFR mutants useful for the therapeutic and/or prophylactic treatment of cancer, in particular T790M and C797S containing EGFR mutants. WO2009158369 describes certain heterocyclic antibacterial agents. WO2016183534 describes certain heterocyclic compounds suitable as EBNA1 inhibitors. WO2011128279 describes certain heterocyclic compounds suitable as mGluR5 modulators. US2018290975 and WO2018220149 describe certain heteroaromatic compounds which allosterically modulate EGFR.

The term "pharmaceutically acceptable salts" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid and the like, in particular hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein and the like. In addition, these salts may be prepared by addition of an inorganic base or an organic base to the free acid. Salts derived from an inorganic base include, but are not limited to, the sodium, potassium, lithium, ammonium, calcium, magnesium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyimine resins and the like. Particular pharmaceutically acceptable salts of compounds of the present invention are the hydrochloride salts, methanesulfonic acid salts and citric acid salts.

The abbreviation uM means microMolar and is equivalent to the symbol µM.

The abbreviation uL means microliter and is equivalent to the symbol µL.

The abbreviation ug means microgram and is equivalent to the symbol µg.

The compounds of the present invention can contain several asymmetric centers and can be present in the form of optically pure enantiomers, mixtures of enantiomers such as, for example, racemates, optically pure diastereoisomers, mixtures of diastereoisomers, diastereoisomeric racemates or mixtures of diastereoisomeric racemates.

According to the Cahn-Ingold-Prelog Convention the asymmetric carbon atom can be of the "R" or "S" configuration.

Also an embodiment of the present invention is a compound as described herein and pharmaceutically acceptable salts thereof, more particularly a compound as described herein.

In another embodiment a compound as described herein is selected from
2-(6,7-dihydro-5H-pyrrolo[1,2-c]imidazol-1-yl)-2-[6-[4-(1-ethyl-4-piperidyl)phenyl]-1-oxo-4-(trifluoromethyl)isoindolin-2-yl]-N-thiazol-2-yl-acetamide;
2-(6,7-dihydro-5H-pyrrolo[1,2-c]imidazol-1-yl)-2-[6-[2-[4-[[4-(hydroxymethyl)-1-piperidyl]methyl]phenyl]ethynyl]-1-oxo-4-(trifluoromethyl)isoindolin-2-yl]-N-thiazol-2-yl-acetamide;
2-[4-(difluoromethyl)-6-[4-(1-ethyl-4-piperidyl)phenyl]-1-oxo-isoindolin-2-yl]-2-(6,7-dihydro-5H-pyrrolo[1,2-c]imidazol-1-yl)-N-thiazol-2-yl-acetamide;
2-[4-(difluoromethyl)-6-[2-[4-[[4-(hydroxymethyl)-1-piperidyl]methyl]phenyl]ethynyl]-1-oxo-isoindolin-2-yl]-2-(6,7-dihydro-5H-pyrrolo[1,2-c]imidazol-1-yl)-N-thiazol-2-yl-acetamide;
2-[6- [2- [4-[[4-(hydroxymethyl)-1-piperidyl]methyl]phenyl]ethynyl] -1 -oxo-4-(trifluoromethyl)isoindolin-2-yl]-2-[rac-(6R)-6-fluoro-6,7-dihydro-5H-pyrrolo[1,2-c]imidazol-1-yl]-N-thiazol-2-yl-acetamide;
2-[4-(difluoromethyl)-6-[4-(1 -ethyl-4-piperidyl)phenyl] -1 -oxo-isoindolin-2-yl] -2-[rac-(6R)-6-fluoro-6,7-dihydro-5H-pyrrolo[1,2-c]imidazol-1-yl]-N-thiazol-2-yl-acetamide;
2-[4-(difluoromethyl)-1-oxo-6-[4-[rac-(3R,4R)-1-ethyl-3-fluoro-4-piperidyl]phenyl]isoindolin-2-yl]-2-(6,7-dihydro-5H-pyrrolo[1,2-c]imidazol-1-yl)-N-thiazol-2-yl-acetamide;
2-[6-[2-[4-[(6-hydroxy-2-azaspiro[3.3]heptan-2-yl)methyl]phenyl]ethynyl]-1-oxo-4-(trifluoromethyl)isoindolin-2-yl]-2-[rac-(6R)-6-fluoro-6,7-dihydro-5H-pyrrolo[1,2-c]imidazol-1-yl]-N-thiazol-2-yl-acetamide;
2-[4-(difluoromethyl)-6-[2-[4-[[4-(hydroxymethyl)-1-piperidyl]methyl]phenyl]ethynyl]-1-oxo-isoindolin-2-yl]-2-[rac-(6R)-6-fluoro-6,7-dihydro-5H-pyrrolo[1,2-c]imidazol-1-yl]-N-thiazol-2-yl-acetamide;
2-[6-[2-[4-[(4-hydroxyazepan-1-yl)methyl]phenyl] ethynyl]-1-oxo-4-(trifluoromethyl)isoindolin-2-yl]-2-[rac-(6R)-6-fluoro-6,7-dihydro-5H-pyrrolo[1,2-c]imidazol-1-yl]-N-thiazol-2-yl-acetamide.

Processes for the manufacture of a compound of the present invention as described herein are also an object of the invention.

The corresponding pharmaceutically acceptable salts with acids can be obtained by standard methods known to the person skilled in the art, e.g. by dissolving the compound of the present invention in a suitable solvent such as e.g. dioxane or tetrahydrofuran and adding an appropriate amount of the corresponding acid. The products can usually be isolated by filtration or by chromatography. The conversion of a compound of the present invention into a pharmaceutically acceptable salt with a base can be carried out by treatment of such a compound with such a base. One possible method to form such a salt is e.g. by addition of 1/n equivalents of a basic salt such as e.g. M(OH)ₙ, wherein M = metal or ammonium cation and n = number of hydroxide anions, to a solution of the compound in a suitable solvent (e.g. ethanol, ethanol-water mixture, tetrahydrofuran-water mixture) and to remove the solvent by evaporation or lyophilisation. Particular salts are hydrochloride, formate and trifluoroacetate.

Insofar as their preparation is not described in the examples, the compounds of the present invention as well as all intermediate products can be prepared according to analogous methods or according to the methods set forth herein. Starting materials are commercially available, known in the art or can be prepared by methods known in the art or in analogy thereto.

It will be appreciated that the compounds of the present invention may be derivatised at functional groups to provide derivatives which are capable of conversion back to the parent compound *in vivo.*

A certain embodiment of the invention relates to the compound of the present invention as described herein, or a pharmaceutically acceptable salt thereof, for use as therapeutically active substance.

A certain embodiment of the invention relates to the compound of the present invention as described herein, or a pharmaceutically acceptable salt thereof, for the use in the therapeutic and/or prophylactic treatment of cancer, in particular non-small-cell lung cancer.

A certain embodiment of the invention relates to the compound of the present invention as described herein, or a pharmaceutically acceptable salt thereof, for the use in the therapeutic and/or prophylactic treatment of non-small-cell lung cancer.

A certain embodiment of the invention relates to the compound of the present invention as described herein, or a pharmaceutically acceptable salt thereof, for the manufacture of a medicament for the therapeutic and/or prophylactic treatment of cancer, in particular non-small-cell lung cancer.

A certain embodiment of the invention relates to a pharmaceutical composition comprising the compound of the present invention as described herein, or a pharmaceutically acceptable salt thereof, and a pharmaceutically acceptable auxiliary substance.

A certain embodiment of the invention relates to the compound of the present invention as described herein, or a pharmaceutically acceptable salt thereof, for the use as a medicament in therapeutic and/or prophylactic treatment of a patient with EGFR activating mutations suffering from cancer, in particular non-small-cell lung cancer, comprising determining the EGFR activating mutations status in said patient and then administering the compound of the present invention as described herein, or a pharmaceutically acceptable salt thereof, to said patient.

A certain embodiment of the invention relates to the compound of the present invention as described herein, or a pharmaceutically acceptable salt thereof, for the use as a medicament in therapeutic and/or prophylactic treatment of a patient with EGFR mutations T790M/L858R, T790M/L858R/C797S, L858R and/or L858R/C797S suffering from cancer, in particular non-small-cell lung cancer, comprising determining the EGFR activating mutations status in said patient and then administering the compound of the present invention as described herein, or a pharmaceutically acceptable salt thereof, to said patient.

A certain embodiment of the invention relates to the compound of the present invention as described herein, or a pharmaceutically acceptable salt thereof, for the use as a medicament in therapeutic and/or prophylactic treatment of a patient with EGFR activating mutations as determined with a cobas^{®} EGFR Mutation Test v2 suffering from cancer, in particular non-small-cell lung cancer, comprising determining the EGFR activating mutations status in said patient and then administering the compound of the present invention as described herein, or a pharmaceutically acceptable salt thereof, to said patient.

Furthermore, the invention includes all substituents in its corresponding deuterated form, wherever applicable, of the compounds of the present invention.

Furthermore, the invention includes all optical isomers, i.e. diastereoisomers, diastereomeric mixtures, racemic mixtures, all their corresponding enantiomers and/or tautomers as well as their solvates, wherever applicable, of the compounds of the present invention.

The compounds of the present invention may contain one or more asymmetric centers and can therefore occur as racemates, racemic mixtures, single enantiomers, diastereomeric mixtures and individual diastereomers. Additional asymmetric centers may be present depending upon the nature of the various substituents on the molecule. Each such asymmetric center will independently produce two optical isomers and it is intended that all of the possible optical isomers and diastereomers in mixtures and as pure or partially purified compounds are included within this invention. The present invention is meant to encompass all such isomeric forms of these compounds. The independent syntheses of these diastereomers or their chromatographic separations may be achieved as known in the art by appropriate modification of the methodology disclosed herein. Their absolute stereochemistry may be determined by the x-ray crystallography of crystalline products or crystalline intermediates which are derivatized, if necessary, with a reagent containing an asymmetric center of known absolute configuration. If desired, racemic mixtures of the compounds may be separated so that the individual enantiomers are isolated. The separation can be carried out by methods well known in the art, such as the coupling of a racemic mixture of compounds to an enantiomerically pure compound to form a diastereomeric mixture, followed by separation of the individual diastereomers by standard methods, such as fractional crystallization or chromatography.

In the embodiments, where optically pure enantiomers are provided, optically pure enantiomer means that the compound contains > 90 % of the desired isomer by weight, particularly > 95 % of the desired isomer by weight, or more particularly > 99 % of the desired isomer by weight, said weight percent based upon the total weight of the isomer(s) of the compound. Chirally pure or chirally enriched compounds may be prepared by chirally selective synthesis or by separation of enantiomers. The separation of enantiomers may be carried out on the final product or alternatively on a suitable intermediate.

Also an embodiment of the present invention are compounds of the present invention as described herein, when manufactured according to any one of the described processes.

### Assay procedures

### HTRF Phospho EGFR TMLRCS assay (cellular)

### Cell line and media

BaF3-TMLRCS cell line were obtained from Crownbio (San Diego, CA, USA). Cells were maintained at 37°C, 5% CO₂ in RPMI ATCC (Gibco 31870) + 2mM Glutamine + 0.5µg/ml Puromycin supplemented with 10% fetal bovine serum (FBS) (Gibco).

### Protocol

Cells are transferred as above to Greiner Bio-One, Nr. 784-08 micro-titerplate at 20000 cells/well in 12.5 µl of growth medium/well after the plates had been pre-filled with 12.5 nl of DMSO solutions of the to be tested compounds (in dose response) or DMSO only. After spinning the plates at 300 x g for 30 seconds the cells were incubated for 4 hours at 37C, 5% CO2, 95% humidity. The cells were lysed by adding to the compound mix 4 µl/well of the supplemented lysis buffer (Cis-bio, Phospho-EGFR HTRF kit, 64EG1PEH), followed by incubation for 30 min at room temperature with shaking (400 rpm). The plates were then frozen and stored overnight at -80C. On the next day and after thawing the plates, 4 µl of a mixture of anti-Phospho-EGFR Cryptate and of anti-Phospho-EGFR-d2 antibody solutions prepared in the supplied detection buffer was added to each well. The lidded plates were then incubated for 4 h at room temperature before reading the fluorescence emission at 616 and 665 nm using an Envision reader (Perkin Elmer). Data was analyzed in similar fashion as above using the normalized ratio of the 665 to 616 signals multiplied by 10000.

The results are shown in Table 1

| **Exam.** | **Structure** | **IC₅₀** (BaF3 TMLRCS) |
|---|---|---|
| 2 | 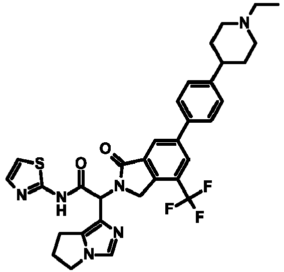 | 20nM |
| 3 | 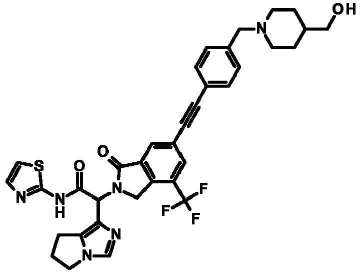 | 1nM |
| 4 | 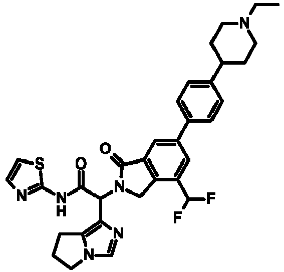 | 11nM |
| 5 | 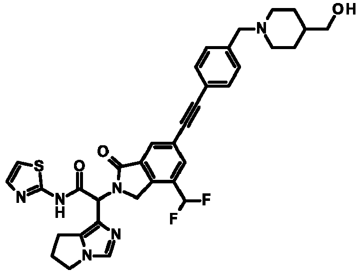 | 3nM |
| 6 | 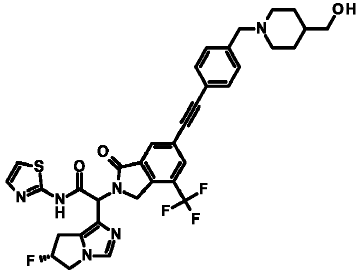 | 1nM |
| 7 | 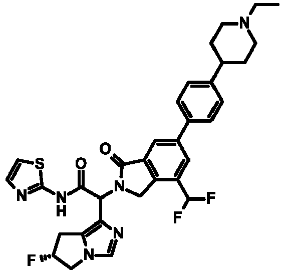 | 16nM |
| 8 | 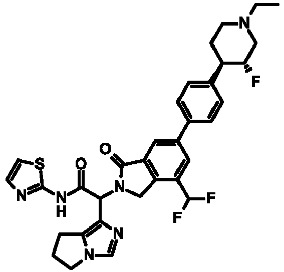 | 18nM |
| 9 | 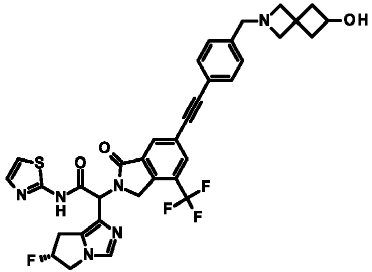 | 3nM |
| 10 | 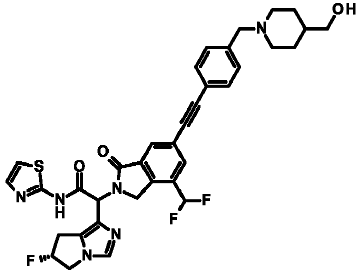 | 2nM |
| 11 | 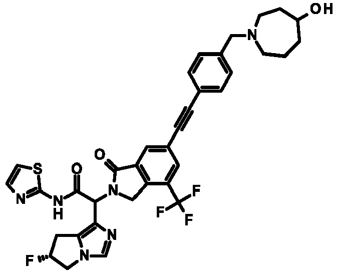 | 2nM |

The compounds of the present invention and their pharmaceutically acceptable salts can be used as medicaments (e.g. in the form of pharmaceutical preparations). The pharmaceutical preparations can be administered internally, such as orally (e.g. in the form of tablets, coated tablets, dragées, hard and soft gelatin capsules, solutions, emulsions or suspensions), nasally (e.g. in the form of nasal sprays), rectally (e.g. in the form of suppositories) or topical ocularly (e.g. in the form of solutions, ointments, gels or water soluble polymeric inserts). However, the administration can also be effected parenterally, such as intramuscularly, intravenously, or intraocularly (e.g. in the form of sterile injection solutions).

The compounds of the present invention and their pharmaceutically acceptable salts can be processed with pharmaceutically inert, inorganic or organic adjuvants for the production of tablets, coated tablets, dragées, hard gelatin capsules, injection solutions or topical formulations Lactose, corn starch or derivatives thereof, talc, stearic acid or its salts etc. can be used, for example, as such adjuvants for tablets, dragées and hard gelatin capsules.

Suitable adjuvants for soft gelatin capsules, are, for example, vegetable oils, waxes, fats, semi-solid substances and liquid polyols, etc.

Suitable adjuvants for the production of solutions and syrups are, for example, water, polyols, saccharose, invert sugar, glucose, etc.

Suitable adjuvants for injection solutions are, for example, water, alcohols, polyols, glycerol, vegetable oils, etc.

Suitable adjuvants for suppositories are, for example, natural or hardened oils, waxes, fats, semi-solid or liquid polyols, etc.

Suitable adjuvants for topical ocular formulations are, for example, cyclodextrins, mannitol or many other carriers and excipients known in the art.

Moreover, the pharmaceutical preparations can contain preservatives, solubilizers, viscosity-increasing substances, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary in wide limits and will, of course, be fitted to the individual requirements in each particular case. In general, in the case of oral administration a daily dosage of about 0.1 mg to 20 mg per kg body weight, preferably about 0.5 mg to 4 mg per kg body weight (e.g. about 300 mg per person), divided into preferably 1-3 individual doses, which can consist, for example, of the same amounts, should it be appropriate. In the case of topical administration, the formulation can contain 0.001% to 15% by weight of medicament and the required dose, which can be between 0.1 and 25 mg in can be administered either by single dose per day or per week, or by multiple doses (2 to 4) per day, or by multiple doses per week It will, however, be clear that the upper or lower limit given herein can be exceeded when this is shown to be indicated.

### Preparation of pharmaceutical compositions comprising compounds of the invention:

Tablets of the following composition are manufactured in the usual manner:

| **Ingredient** | **mg/tablet** | | | |
|---|---|---|---|---|
| | 5 | 25 | 100 | 500 |
| Compound | 5 | 25 | 100 | 500 |
| Lactose Anhydrous DTG | 125 | 105 | 30 | 150 |
| Sta-Rx 1500 | 6 | 6 | 6 | 60 |
| Microcrystalline Cellulose | 30 | 30 | 30 | 450 |
| Magnesium Stearate | 1 | 1 | 1 | 1 |
| Total | 167 | 167 | 167 | 831 |

### Manufacturing Procedure

1. Mix ingredients 1, 2, 3 and 4 and granulate with purified water.
2. Dry the granules at 50°C.
3. Pass the granules through suitable milling equipment.
4. Add ingredient 5 and mix for three minutes; compress on a suitable press.

Capsules of the following composition are manufactured:

| **Ingredient** | **mg/capsule** | | | |
|---|---|---|---|---|
| | 5 | 25 | 100 | 500 |
| Compound | 5 | 25 | 100 | 500 |
| Hydrous Lactose | 159 | 123 | 148 | - |
| Corn Starch | 25 | 35 | 40 | 70 |
| Talk | 10 | 15 | 10 | 25 |
| Magnesium Stearate | 1 | 2 | 2 | 5 |
| Total | 200 | 200 | 300 | 600 |

### Manufacturing Procedure

1. Mix ingredients 1, 2 and 3 in a suitable mixer for 30 minutes.
2. Add ingredients 4 and 5 and mix for 3 minutes.
3. Fill into a suitable capsule.

A compound of the present invention, lactose and corn starch are firstly mixed in a mixer and then in a comminuting machine. The mixture is returned to the mixer; the talc is added thereto and mixed thoapproximatively. The mixture is filled by machine into suitable capsules, e.g. hard gelatin capsules.

Injection solutions of the following composition are manufactured:

| **Ingredient** | **mg/injection solution.** |
|---|---|
| Compound | 3 |
| Polyethylene Glycol 400 | 150 |
| acetic acid | q.s. ad pH 5.0 |
| water for injection solutions | ad 1.0 ml |

The invention is illustrated hereinafter by Examples, which have no limiting character.

In case the preparative examples are obtained as a mixture of enantiomers, the pure enantiomers can be obtained by methods described herein or by methods known to those skilled in the art, such as e.g. chiral chromatography or crystallization.

### Experimental Part

### General synthesis using Reference compound 1

### (2RS)-2-(6,7-Dihydro-5H-pyrrolo[1,2-c]imidazol-1-yl)-2-[6-[2-[4-[(4-hydroxy-1-piperidyl)methyl]phenyl]ethynyl]-1-oxo-4-(trifluoromethyl)isoindolin-2-yl]-N-thiazol-2-yl-acetamide

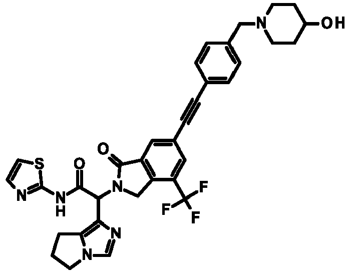

### Step 1: Ethyl 2-(6,7-dihydro-5H-pyrrolo[1,2-c]imidazol-1-yl)-2-oxo-acetate

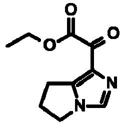

To a solution of ethyl 2-(6,7-dihydro-5H-pyrrolo[1,2-c]imidazol-1-yl)acetate (20.0 g, 102.97 mmol) dissolved in 200 ml of 1,4-dioxane was added selenium dioxide (22.85 g, 205.94 mmol, 2 equiv.). The reaction mixture was stirred for 5 hours at 80°C. The reaction mixture was concentrated under vacuum to give a residue. The crude product was purified by flash chromatography on a silica gel column eluting with petroleum ether:ethyl acetate 2:1 to ethyl acetate:ethanol 10:1 gradient to obtain the desired ethyl 2-(6,7-dihydro-5H-pyrrolo[1,2-c]imidazol-1-yl)-2-oxo-acetate (quant. yield) as a light brown oil, MS: m/e = 209.1 (M+H⁺).

### Step 2: Ethyl 2-(6,7-dihydro-5H-pyrrolo[1,2-c]imidazol-1-yl)-2-hydroxyimino-acetate

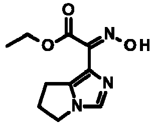

To a solution of ethyl 2-(6,7-dihydro-5H-pyrrolo[1,2-c]imidazol-1-yl)-2-oxo-acetate *(Reference compound 1, step 1)* (17.5 g, 84.05 mmol) dissolved in 145 ml of ethanol was added hydroxylamine hydrochloride (6.42 g, 92.45 mmol, 1.1 equiv.) and sodium acetate (13.79 g, 168.1 mmol, 2 equiv.) at room temperature. The reaction mixture was stirred for 3.5 hours at 80°C. The reaction mixture was concentrated and extracted with water and five times with a mixture of ethanol/THF/ethyl acetate 1:1:8. The organic layers were concentrated to dryness. The desired ethyl 2-(6,7-dihydro-5H-pyrrolo[1,2-c]imidazol-1-yl)-2-hydroxyimino-acetate (15 g, 80 % yield) was obtained as a yellow solid, MS: m/e = 224.1 (M+H⁺) and used directly in the next step.

### Step 3: Ethyl (2RS)-2-amino-2-(6,7-dihydro-5H-pyrrolo[1,2-c]imidazol-1-yl)acetate

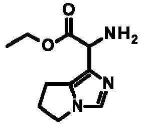

To a solution of ethyl 2-(6,7-dihydro-5H-pyrrolo[1,2-c]imidazol-1-yl)-2-hydroxyimino-acetate *(Reference compound 1, step 2)* (15.0 g, 67.2 mmol) dissolved in 225 ml of ethanol and 120 ml of THF was added Pd/C (30.0g, 67.2 mmol, 1 eq, 10%) at room temperature. The mixture was hydogenated with H₂ for 24 hours at 45°C. The reaction mixture was filtered and the filtrate was concentrated under vacuum. The desired ethyl (2*RS*)-2-amino-2-(6,7-dihydro-5H-pyrrolo[1,2-c]imidazol-1-yl)acetate (quant. yield) was obtained as a brown oil, MS: m/e = 210.1 (M+H⁺) and used directly in the next step.

### Step 4: Ethyl (2RS)-2-amino-2-(6,7-dihydro-5H-pyrrolo[1,2-c]imidazol-1-yl)acetate hydrochloride

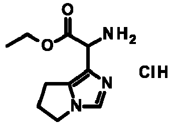

A solution of ethyl (2*RS*)-2-amino-2-(6,7-dihydro-5H-pyrrolo[1,2-c]imidazol-1-yl)acetate *(Reference compound 1, step 3)* (15.0 g, 82.79 mmol) in HCl/EtOH (300 ml, 1200 mmol, 14.5 equiv., 2.5 mol/L) was stirred at 25 °C for 36 hours. The reaction mixture was concentrated under vacuum below 25°C to give a residue as brown oil. 150 ml of acetonitrile were added to the residue and the precipitated yellow solid was collected and dried under vacuum below 25 °C to give the desired ethyl (2*RS*)-2-amino-2-(6,7-dihydro-5H-pyrrolo[1,2-c]imidazol-1-yl)acetate hydrochloride (quant. yield) as yellow solid, MS: m/e = 210.1 (M+H⁺).

### Step 5: 5-lodo-2-methyl-3-(trifluoromethyl)benzoic acid

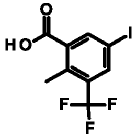

2-Methyl-3-(trifluoromethyl)benzoic acid (3.4 g, 16.9 mmol) was dissolved in 20 ml of sulfuric acid. 1,3-Diiodo-5,5-dimethylimidazolidine-2,4-dione (3.35 g, 8.82 mmol, 0.52 equiv.) was added at room temperature. The mixture was stirred at room temperature for 1 hour. The reaction mixture was poured onto water and the resulting precipitate filtered off. The solid was dried to obtain the desired product (5.6 g, quant. yield) as a light yellow solid, MS: m/e = 329.1 (M-H+).

### Step 6: Methyl 5-iodo-2-methyl-3-(trifluoromethyl)benzoate

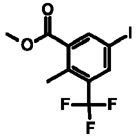

5-Iodo-2-methyl-3-(trifluoromethyl)benzoic acid (5.6 g, 16.6 mmol) was dissolved in 40 ml of DMF. Potassium carbonate (4.6 g, 33.3 mmol, 2 equiv.) and iodomethane (1.09 ml, 2.48 g, 17.5 mmol, 1.05 equiv.) were added at room temperature. The mixture was stirred for 2.5 hours. The reaction mixture was extracted with ethyl acetate and saturated NaHCO3-solution. The aqueous layer was back-extracted with ethyl acetate. The organic layers were washed with water. The organic layers were combined, dried over sodium sulfate, filtered and concentrated to dryness. The crude product was purified by flash chromatography on a silica gel column eluting with an ethyl acetate:heptane 0:100 to 50:50 gradient. The desired product (4.5 g, 71 % yield) was obtained as a white solid.

### Step 7: Methyl 2-(bromomethyl)-5-iodo-3-(trifluoromethyl)benzoate

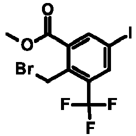

Methyl 5-iodo-2-methyl-3-(trifluoromethyl)benzoate *(Reference compound 1, step 6)* (4.8 g, 11.8 mmol) was dissolved in 60 ml trifluorotoluene and N-bromosuccinimide (2.34 g, 13.1 mmol, 1 equiv.) and AIBN (200 mg, 1.2 mmol, 0.1 equiv.) were added at room temperature. The mixture was stirred at 110°C for 3 hours. The reaction mixture was cooled, extracted with water and two times with ethyl acetate. The organic layers were dried over sodium sulfate and concentrated to dryness. The crude product was purified by flash chromatography on a silica gel column eluting with an ethyl acetate:heptane 0:100 to 30:70 gradient to obtain the desired product (4.94 g, 75 % purity, 75 % yield) as a colorless oil.

### Step 8: Ethyl (2RS)-2-(6,7-dihydro-5H-pyrrolo[1,2-c]imidazol-1-yl)-2-[6-iodo-1-oxo-4-(trifluoromethyl)isoindolin-2-yl]acetate

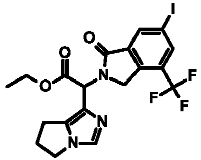

Ethyl (2*RS*)-2-amino-2-(6,7-dihydro-5H-pyrrolo[1,2-c]imidazol-1-yl)acetate hydrochloride *(Reference compound 1, step 4)* (930 mg, 3.78 mmol, 1 equiv.) was dissolved in 1.5 ml of DMF. Methyl 2-(bromomethyl)-5-iodo-3-(trifluoromethyl)benzoate *(Reference compound 1, step* 7) (1.6 g, 3.78 mmol) and triethylamine (1.6 ml, 11.3 mmol, 3 equiv.) were added at room temperature. The mixture was stirred at room temperature for 30 minutes and at 100°C for 1 hour. The reaction mixture was extracted with water and two times with ethyl acetate. The organic layers were extracted with brine, dried over sodium sulfate and concentrated to dryness. The crude product was purified by flash chromatography on a silica gel column eluting with a dichloromethane:methanol 100:0 to 90:10 gradient to obtain the desired product (1.13 g, 56 % yield) as a dark brown oil, MS: m/e = 520.0 (M+H⁺).

### Step 9: (2RS)-2-(6,7-Dihydro-5H-pyrrolo[1,2-c]imidazol-1-yl)-2-[6-iodo-1-oxo-4-(trifluoromethyl)isoindolin-2-yl]-N-thiazol-2-yl-acetamide

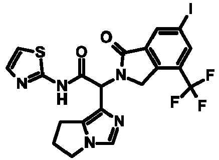

Ethyl (2RS)-2-(6,7-dihydro-5H-pyrrolo[1,2-c]imidazol-1-yl)-2-[6-iodo-1-oxo-4-(trifluoromethyl)isoindolin-2-yl]acetate *(Reference compound 1, step 8)* (550 mg, 1.06 mmol) was dissolved in 20 ml of methanol and 20 ml of THF. LiOH (1M in water) (1.27 ml, 1.27 mmol, 1.2 equiv.) was added at room temperature. The mixture was stirred for 1 hour at room temperature. The reaction mixture was concentrated in vacuo and the residue was dissolved in 20 ml of DMF. Thiazol-2-amine (138 mg, 1.38 mmol, 1.3 equiv.), Hunig's base (0.92 ml, 5.3 mmol, 5 equiv.) and HATU (480 mg, 1.27 mmol, 1.2 equiv.) were added at room temperature. The mixture was stirred at room temperature for 30 minutes. The reaction mixture was extracted with water and two times with ethyl acetate. The organic layers were extracted with water, dried over sodium sulfate and concentrated to dryness. The crude product was purified by flash chromatography on a silica gel column eluting with a dichloromethane:methanol 100:0 to 90: 10 gradient to obtain the desired product (420 mg, 68 % yield) as an orange semi-solid, MS: m/e = 574.0 (M+H⁺).

### Step 10: 1-[(4-Ethynylphenyl)methyl]piperidin-4-ol

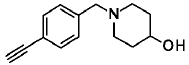

4-Ethynylbenzaldehyde (2.8 g, 21.5 mmol) was dissolved in 85 ml of dichloromethane. Piperidin-4-ol (2.1 g, 21.5 mmol, 1.0 equiv.) and sodium triacetoxyborohydride (7.75 g, 36.6 mmol, 1.7 equiv.) were added at room temperature. The mixture was stirred at room temperature for 5 hours. The reaction mixture was extracted with water and two times with dichloromethane. The organic layers were extracted with brine, dried over sodium sulfate and concentrated to dryness. The crude product was purified by flash chromatography on a silica gel column eluting with a dichloromethane:methanol 100:0 to 80:20 gradient to obtain the desired product (3.06 g, 66 % yield) as a light yellow solid, MS: m/e = 216.3 (M+H⁺).

### Step 11: (2RS)-2-(6,7-Dihydro-5H-pyrrolo[1,2-c]imidazol-1-yl)-2-[6-[2-[4-[(4-hydroxy-1-piperidyl)methyl]phenyl]ethynyl]-1-oxo-4-(trifluoromethyl)isoindolin-2-yl]-N-thiazol-2-yl-acetamide

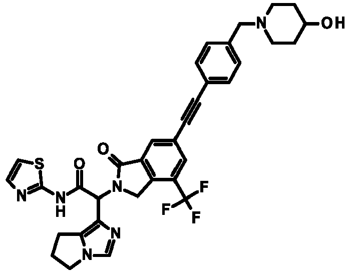

(2RS)-2-(6,7-Dihydro-5H-pyrrolo[1,2-c]imidazol-1-yl)-2-[6-iodo-1-oxo-4-(trifluoromethyl)isoindolin-2-yl]-N-thiazol-2-yl-acetamide *(Reference compound 1, step 9)* (210 mg, 0.37 mmol) and 1-[(4-ethynylphenyl)methyl]piperidin-4-ol *(Reference compound 1, step 10)* (118 mg, 0.55 mmol, 1.5 equiv.) were dissolved in 4 ml of DMF. Triethylamine (111 mg, 0.15 ml, 1.1 mmol, 3 equiv.), bis-(triphenylphosphine)-palladium(II)dichloride (13 mg, 0.018 mmol, 0.05 equiv.), triphenylphosphine (10 mg, 0.04 mmol, 0.1 equiv.) and copper(I)iodide (3 mg, 0.018 mmol, 0.05 equiv.) were added and the mixture was stirred for 2 hours at 80°C. The reaction mixture was extracted with water and two times with ethyl acetate. The organic layers were extracted with brine, dried over sodium sulfate and concentrated to dryness. The crude product was purified by flash chromatography on a silica gel column eluting with a dichloromethane:methanol 100:0 to 90:10 gradient to obtain the desired product (144 mg, 59 % yield) as a white solid, MS: m/e = 661.4 (M+H⁺).

### Example 2

### (2RS)-2-(6,7-Dihydro-5H-pyrrolo [1,2-c] imidazol-1-yl)-2- [6- [4-(1-ethyl-4-piperidyl)phenyl] - 1-oxo-4-(trifluoromethyl)isoindolin-2-yl]-N-thiazol-2-yl-acetamide

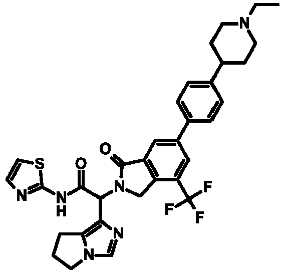

### Step 1:4-(4-Bromophenyl)-1-ethyl-piperidine

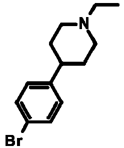

4-(4-Bromophenyl)piperidine (5 g, 20.8 mmol) was dissolved in 25 ml of DMF. Ethyl iodide (3.57 g, 1.85 ml, 22.9 mmol, 1.1 equiv.) and Hunig's base (5.38 g, 7.3 ml, 41.6 mmol, 2 equiv.) were added at room temperature. The reaction mixture was stirred at room temperature for 2 hours. The reaction mixture was cooled to room temperature and then extracted with ethyl acetate and water. The aqueous layer was back-extracted with ethyl acetate. The organic layers were washed with brine. The organic layers were combined, dried over sodium sulfate, filtered and concentrated to dryness. The crude product was purified by flash chromatography on a silica gel column eluting with a ethyl acetate: methanol 100:0 to 80:20 gradient to obtain the desired product (4.6 g, 78 % yield) as a yellow liquid, MS: m/e = 268.1/270.1 (M+H⁺).

### Step 2: 1 -Ethyl-4-[4-(4,4,5,5-tetramethyl-1,3,2-dioxaborolan-2-yl)phenyl]piperidine

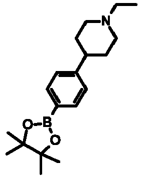

4-(4-Bromophenyl)piperidine *(Example 2, step 1)* (4.6 g, 15.4 mmol, 1.0 equiv.) and bis(pinacolato)diboron (4.31 g, 17 mmol, 1.1 equiv.) were dissolved in 10 ml of dioxane. Potassium acetate (4.54 g, 46.3 mmol, 3.0 equiv.) and dichloro 1,1'-bis(diphenylphosphino)ferrocene palladium(II) dichloromethane adduct (630 mg, 0.77 mmol, 0.05 equiv.) were added and the reaction mixture was stirred at 90°C for 20 hours. The reaction mixture was cooled to room temperature and concentrated to dryness. The crude product was purified by flash chromatography on a silica gel column eluting with a dichloromethane:methanol 100:0 to 80:20 gradient to obtain the desired product (5.6 g, 92 % yield) as a dark brown oil, MS: m/e = 316.2 (M+H⁺).

### Step 3: Ethyl (2RS)-2-(6,7-dihydro-5H-pyrrolo[1,2-c]imidazol-1-yl)-2-[6-[4-(1-ethyl-4-piperidyl)phenyl]-1-oxo-4-(trifluoromethyl)isoindolin-2-yl]acetate

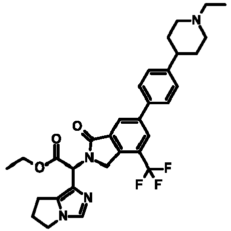

Ethyl (2RS)-2-(6,7-dihydro-5H-pyrrolo[1,2-c]imidazol-1-yl)-2-[6-iodo-1-oxo-4-(trifluoromethyl)isoindolin-2-yl]acetate *(Reference compound 1, step 8)* (150 mg, 0.29 mmol) and 1-ethyl-4-[4-(4,4,5,5-tetramethyl-1,3,2-dioxaborolan-2-yl)phenyl]piperidine *(Example 2, step 2)* (118 mg, 0.37 mmol, 1.3 equiv.) were dissolved in 1.5 ml of THF and 0.25 ml of water. Cesium carbonate (280 mg, 0.87 mmol, 3.0 equiv.) and PdC12(DPPF)-CH2C12 adduct (24 mg, 0.029 mmol, 0.1 equiv.) were added and the reaction mixture was stirred at 60°C for 3 hours. The reaction mixture was cooled to room temperature and then extracted with ethyl acetate and saturated NaHCOs-solution. The aqueous layer was back-extracted with ethyl acetate. The organic layers were washed with water and brine. The organic layers were combined, dried over sodium sulfate, filtered and concentrated to dryness. The crude product was purified by flash chromatography on a silica gel column eluting with a dichloromethane:methanol 100:0 to 90:10 gradient. The desired product (75 mg, 45 % yield) was obtained as a yellow oil, MS: m/e = 581.5 (M+H⁺).

### Step 4: (2RS)-2-(6,7-Dihydro-5H-pyrrolo[1,2-c]imidazol-1-yl)-2-[6-[4-(1-ethyl-4-piperidyl)phenyl]-1-oxo-4-(trifluoromethyl)isoindolin-2-yl]-N-thiazol-2-yl-acetamide

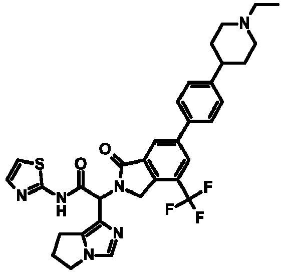

The title compound was obtained as a light brown solid, MS: m/e = 635.6 (M+H⁺), using chemistry similar to that described in Reference compound 1, step 9 starting from ethyl (2RS)-2-(6,7-dihydro-5H-pyrrolo[1,2-c]imidazol-1-yl)-2-[6-[4-(1-ethyl-4-piperidyl)phenyl]-1-oxo-4-(trifluoromethyl)isoindolin-2-yl]acetate *(Example 2, step 3)* and thiazol-2-amine.

### Example 3

### (2RS)-2-(6,7-Dihydro-5H-pyrrolo[1,2-c]imidazol-1-yl)-2-[6-[2-[4-[[4-(hydroxymethyl)-1-piperidyl] methyl] phenyl] ethynyl]-1-oxo-4-(trifluoromethyl)isoindolin-2-yl]-N-thiazol-2-yl-acetamide

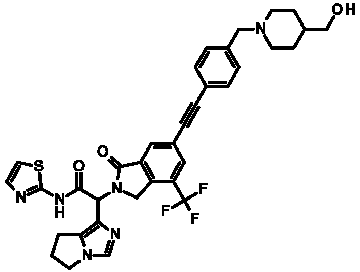

### Step 1: [1-[(4-Ethynylphenyl)methyl]-4-piperidyl]methanol

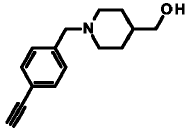

The title compound was obtained as an orange oil, MS: m/e = 230.2 (M+H⁺), using chemistry similar to that described in Reference compound 1, step 10 starting from 4-ethynylbenzaldehyde and piperidin-4-ylmethanol.

### Step 2: (2RS)-2-(6,7-Dihydro-5H-pyrrolo[1,2-c]imidazol-1-yl)-2-[6-[2-[4-[[4-(hydroxymethyl)-1-piperidyl]methyl]phenyl]ethynyl]-1-oxo-4-(trifluoromethyl)isoindolin-2-yl]-N-thiazol-2-yl-acetamide

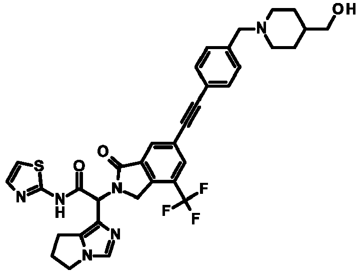

The title compound was obtained as white solid, MS: m/e = 675.4 (M+H⁺), using chemistry similar to that described in Reference compound 1, step 11 starting from (2RS)-2-(6,7-dihydro-5H-pyrrolo[1,2-c]imidazol-1-yl)-2-[6-iodo-1-oxo-4-(trifluoromethyl)isoindolin-2-yl]-N-thiazol-2-yl-acetamide *(Reference compound 1, step 9)* and [1-[(4-ethynylphenyl)methyl]-4-piperidyl]methanol *(Example 3, step 1).*

### Example 4

### (2RS)-2-[4-(Difluoromethyl)-6-[4-(1-ethyl-4-piperidyl)phenyl]-1-oxo-isoindolin-2-yl]-2-(6,7-dihydro-SH-pyrrolo [1,2-c] imidazol-1-yl)-N-thiazol-2-yl-acetamide

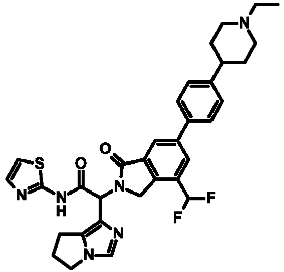

### Step 1: Methyl 5-bromo-3-formyl-2-methyl-benzoate

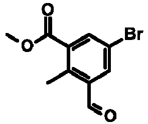

The title compound was obtained as white solid using chemistry similar to that described in Reference compound 1, step 6 starting from methyl 3-formyl-2-methyl-benzoate (CAS 1460037-59-5) and NBS.

### Step 2: Methyl 5-bromo-3-(difluoromethyl)-2-methyl-benzoate

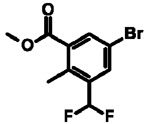

Methyl 5-bromo-3-formyl-2-methyl-benzoate *(Example 4, step 1)* (4.5 g, 17.5 mmol) was dissolved in 180 ml of dichloromethane. DAST (14.1 g, 87.52 mmol, 5.0 equiv.) was added at room 0-5 °C. The mixture was stirred at room temperature for 3 hours. The reaction mixture was concentrated under vacuum to give a residue. The crude product was purified by flash chromatography on a silica gel column eluting with a petroleum ether:ethyl acetate 20:1 to 3:1 gradient. The desired product (3.7 g, 76 % yield) was obtained as a white solid.

### Step 3: Methyl 5-bromo-2-(bromomethyl)-3-(difluoromethyl)benzoate

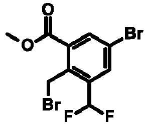

The title compound was obtained as colorless oil using chemistry similar to that described in Reference compound 1, step 7 starting from methyl 5-bromo-3-(difluoromethyl)-2-methylbenzoate *(Example 4, step 2).*

### Step 4: Ethyl (2RS)-2-[6-bromo-4-(difluoromethyl)-1-oxo-isoindolin-2-yl]-2-(6,7-dihydro-5H-pyrrolo[1,2-c]imidazol-1-yl)acetate

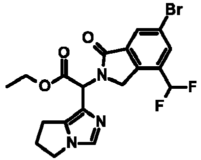

The title compound was obtained as purple solid, MS: m/e = 456.0 (M+H⁺), using chemistry similar to that described in Reference compound 1, step 8 starting from ethyl (2*RS*)-2-amino-2-(6,7-dihydro-5H-pyrrolo[1,2-c]imidazol-1-yl)acetate hydrochloride *(Reference compound 1, step* 4) and methyl 5-bromo-2-(bromomethyl)-3-(difluoromethyl)benzoate *(Example 4, step 3).*

### Step 5: (2RS)-2-[4-(Difluoromethyl)-6-[4-(1-ethyl-4-piperidyl)phenyl]-1-oxo-isoindolin-2-yl]-2-(6,7-dihydro-5H-pyrrolo[1,2-c]imidazol-1-yl)-N-thiazol-2-yl-acetamide

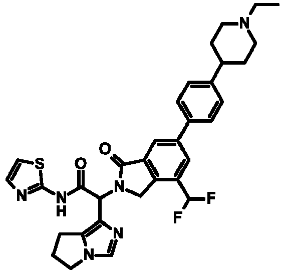

The title compound was obtained as purple solid, MS: m/e = 456.0 (M+H⁺), using chemistry similar to that described in Example 2, step 3 and Reference compound 1, step 9 starting from ethyl (2RS)-2-[6-bromo-4-(difluoromethyl)-1-oxo-isoindolin-2-yl]-2-(6,7-dihydro-5H-pyrrolo[1,2-c]imidazol-1-yl)acetate *(Example 4, step 4)* and 1-ethyl-4-[4-(4,4,5,5-tetramethyl-1,3,2-dioxaborolan-2-yl)phenyl]piperidine *(Example 2, step 2).*

### Example 5

### (2RS)-2-[4-(Difluoromethyl)-6-[2-[4-[[4-(hydroxymethyl)-1-piperidyl] methyl] phenyl]ethynyl]-1-oxo-isoindolin-2-yl]-2-(6,7-dihydro-5H-pyrrolo[1,2-c]imidazol-1-yl)-N-thiazol-2-yl-acetamide

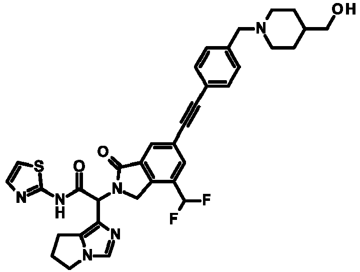

### Step 1: (2RS)-2-[6-Bromo-4-(difluoromethyl)-1-oxo-isoindolin-2-yl]-2-(6,7-dihydro-5H-pyrrolo[1,2-c]imidazol-1-yl)-N-thiazol-2-yl-acetamide

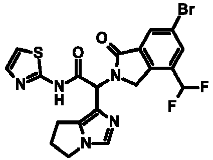

The title compound was obtained as a yellow solid, MS: m/e = 510.1 (M+H⁺), using chemistry similar to that described in Reference compound 1, step 9 starting from ethyl (2RS)-2-[6-bromo-4-(difluoromethyl)-1 -oxo-isoindolin-2-yl]-2-(6,7-dihydro-5H-pyrrolo[1,2-c]imidazol-1-yl)acetate *(Example 4, step 4)* and thiazol-2-amine.

### Step 2: (2RS)-2-[4-(Difluoromethyl)-6-[2-[4-[[4-(hydroxymethyl)-1-piperidyl]methyl]phenyl]ethynyl]-1-oxo-isoindolin-2-yl]-2-(6,7-dihydro-5H-pyrrolo[1,2-c]imidazol-1-yl)-N-thiazol-2-yl-acetamide

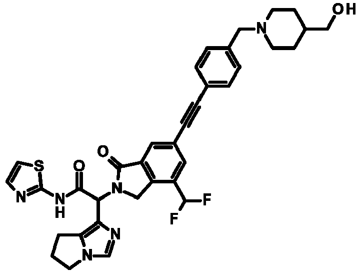

The title compound was obtained as white solid, MS: m/e = 657.4 (M+H⁺), using chemistry similar to that described in Reference compound 1, step 11 starting from (2RS)-2-[6-bromo-4-(difluoromethyl)-1-oxo-isoindolin-2-yl]-2-(6,7-dihydro-5H-pyrrolo[1,2-c]imidazol-1-yl)-N-thiazol-2-yl-acetamide *(Example 5, step 1)* and [1-[(4-ethynylphenyl)methyl]-4-piperidyl]methanol *(Example 3, step 1).*

### Example 6

### (2RS)-2-[(6R)-6-Fluoro-6,7-dihydro-SH-pyrrolo [1,2-c] imidazol-1-yl]-2-[6-[2-[4-[[4-(hydroxymethyl)-1-piperidyl]methyl]phenyl]ethynyl]-1-oxo-4-(trifluoromethyl)isoindolin-2-yl]-N-thiazol-2-yl-acetamide

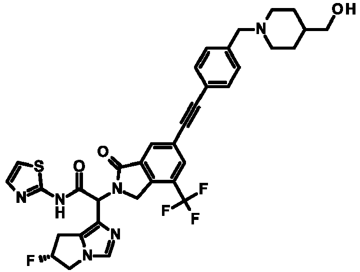

### Step 1: tert-Butyl (2RS,4R)-2-(2,2-dimethyl-4,6-dioxo-E3-dioxane-5-carbonyl)-4-fluoro-pyrrolidine-1 -carboxylate

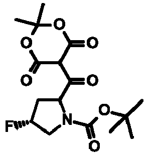

To a solution of (2S,4R)-1-tert-butoxycarbonyl-4-fluoro-pyrrolidine-2-carboxylic acid (450.0 g, 1.93 mol, 1.00 eq.) and 2,2-dimethyl-1,3-dioxane-4,6-dione (278.0 g, 1.93 mol, 1.00eq.) in dichloromethane (3.00 L) was added DMAP (471.4 g, 3.86 mol, 2.00 eq.) and the mixture was cooled down to 0 °C. DCC (398.0 g, 1.93 mol, 390.3 mL, 1.00 eq.) was added in several portions and the reaction mixture was stirred at 25 °C for 12 hours. The reaction mixture was filtered and then the filtrate was washed with 1M HCl (2.00 L) and then organic layer were concentrated in vacuum to give the desired product (1.20 kg, 3.34 mol, 86.5% yield) as a yellow solid used directly for next step.

### Step 2: tert-Butyl (2RS,4R)-2-(3-ethoxy-3-oxo-propanoyl)-4-fluoro-pyrrolidine-1-carboxylate

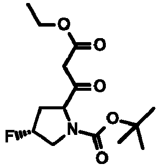

A mixture of tert-butyl (2RS,4R)-2-(2,2-dimethyl-4,6-dioxo-1,3-dioxane-5-carbonyl)-4-fluoro-pyrrolidine-1-carboxylate *(Example 6, step 1)* (650.0 g, 1.81 mol, 1.00 eq.) in EtOH (2.00 L) was stirred at 95 °C for 12 hours. The reaction mixture was concentrated under reduced pressure to yield the desired product as a light yellow oil (1.00 kg, 3.30 mol, 91.1% yield) which was used for next step directly.

### Step 3: Ethyl 3-r(2RS,4R)-4-fluoropyrrolidin-2-yl1-3-oxo-propanoate hydrochloride

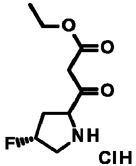

A mixture of tert-butyl (2RS,4R)-2-(3-ethoxy-3-oxo-propanoyl)-4-fluoro-pyrrolidine-1-carboxylate (320.0 g, 1.05 mol, 1.00 eq.) and HCl/EtOAc (4.00 M, 800.0 mL, 3.03 eq.) was stirred at 25 °C for 2 hours. The reaction mixture was concentrated under reduced pressure to give the desired product (603.0 g, 2.97 mol, 93.8% yield) as a red oil that was used directly for next reaction without further purification.

### Step 4: Ethyl 2-[(6R)-6-fluoro-3-thioxo-2,5,6,7-tetrahydropyrrolo[1,2-c]imidazol-1-yl]acetate

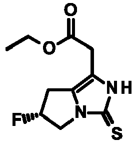

To a solution of ethyl 3-[(2RS,4R)-4-fluoropyrrolidin-2-yl]-3-oxo-propanoate hydrochloride (200.0 g, 834.5 mmol, 1.00 eq., HCl) in EtOH (1.20 L) , KSCN (89.20 g,917.9 mmol, 89.2 mL, 1.10 eq.) was added and the mixture was heated to 90 °C and stirred for 12hours. The mixture was concentrated in vacuum to give a crude solid. The crude solid was washed with EtOAc (5.00 L) and H2O (3.00 L) and concentrated in vacuum to give compound 5 (555.0 g, 2.27 mol, 90.7% yield) as a white solid.

### Step 5: Ethyl 2-[(6R)-6-fluoro-6,7-dihydro-5H-pyrrolo[1,2-c]imidazol-1-yl]acetate

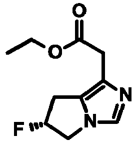

A mixture of ethyl 2-[(6R)-6-fluoro-3-thioxo-2,5,6,7-tetrahydropyrrolo[1,2-c]imidazol-1-yl]acetate (285.0 g, 1.17 mol, 1.00 eq ) in AcOH (1.14 L) was added H2O2 (529.1 g, 4.67 mol, 448.4 mL, 30% purity, 4.00 eq. ) dropwised at 0-25°C. The reaction mixture was stirred at 25 °C for 30 minutes. The reaction mixture was diluted with water (4.00 L) and then adjust to pH = 9 by addition of solid NaHCO3 at 0°C. The mixture was extracted six times with DCM (1.00 L each). The combined organic layers were washed with saturated NaCl solution (3.00 L) and the combined organic layers washed three times with saturated Na2S2O3 solution (2.00 L each). The organic layer was dried over Na2SO4, filtered and concentrated under reduced pressure to give the desired product (156.0 g, crude) as a brown oil.

### Step 6: Ethyl 2-[(6R)-6-fluoro-6,7-dihydro-5H-pyrrolo[1,2-c]imidazol-1-yl]-2-oxo-acetate

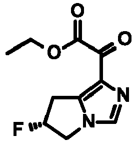

To a solution of ethyl 2-[(6R)-6-fluoro-6,7-dihydro-5H-pyrrolo[1,2-c]imidazol-1-yl]acetate (42.3 g, 199.3 mmol, 1.00 eq.) in dioxane (450.0 mL), SeO2 (44.2 g, 398.6 mmol, 43.3 mL, 2.00 eq.) was added and the mixture was heated to 80 °C and stirred for 2hours. The mixture was added silica gel and concentrated to give a residue which was purified with silica gel chromatography pure EtOAc to give the desired product (54.0 g, 238.7 mmol, 59.8% yield) as a black brown oil.

### Step 7: Ethyl 2-[(6R)-6-fluoro-6,7-dihydro-5H-pyrrolo[1,2-c]imidazol-1-yl]-2-hydroxyimino-acetate

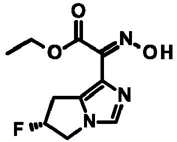

To a solution of ethyl 2-[(6R)-6-fluoro-6,7-dihydro-5H-pyrrolo[1,2-c]imidazol-1-yl]-2-oxo-acetate (18.6 g, 82.2 mmol, 1.00 eq.) in EtOH (90.0 mL) was added NaOAc (13.5 g, 164.5 mmol, 2.00 eq.) and NH2OHHCl (6.30 g, 90.5 mmol, 1.10 eq.) The reaction mixture was stirred at 80 °C for 2hours. The mixture was cooled to 25 °C and filtered then concentrated to give a residue which was diluted with EtOAc: EtOH (20:1)(300.0 mL) and washed with water (200.0 mL) and saturated NaCl solution (200.0 mL), dried with anhydrous Na2SO4 to give the desired product (18.0 g, 74.6 mmol, 90.8% yield) as a black brown oil which was used directly without further purification.

### Step 8: Ethyl (2RS)-2-amino-2-[(6R)-6-fluoro-6,7-dihydro-5H-pyrrolo[1,2-c]imidazol-1-yl]acetate

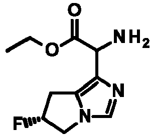

A solution of ethyl 2-[(6R)-6-fluoro-6,7-dihydro-5H-pyrrolo[1,2-c]imidazol-1-yl]-2-hydroxyimino-acetate (18.0 g, 70.9 mmol, 1.00 eq.) and Pd/C (36.0 g, 10% purity) in THF (90.0 mL) and EtOH (180.0 mL) was stirred at 50 °C for 16 h under H2 (15.0 Psi). The mixture was filtered and the filter cake was washed with EtOAc. The filtrate was concentrated to give the desired product (11.0 g, 43.3 mmol, 61.1% yield, 89.5% purity) as a green oil.

### Step 9: Ethyl (2RS)-2-[(6R)-6-fluoro-6,7-dihydro-5H-pyrrolo[1,2-c]imidazol-1-yl]-2-[6-iodo-1-oxo-4-(trifluoromethyl)isoindolin-2-yl]acetate

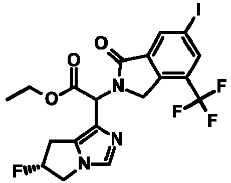

The title compound was obtained as a light brown solid, MS: m/e = 538.1 (M+H⁺), using chemistry similar to that described in Reference compound 1, step 8 starting from ethyl (2RS)-2-amino-2-[(6R)-6-fluoro-6,7-dihydro-5H-pyrrolo[1,2-c]imidazol-1-yl]acetate *(Example 6, step 8)* and methyl 2-(bromomethyl)-5-iodo-3-(trifluoromethyl)benzoate *(Reference compound 1, step 7).*

### Step 10: (2RS)-2-[6-Iodo-1-oxo-4-(trifluoromethyl)isoindolin-2-yl]-2-[(6R)-6-fluoro-6,7-dihydro-5H-pyrrolo[1,2-c]imidazol-1-yl]-N-thiazol-2-yl-acetamide

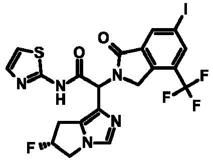

The title compound was obtained as a light brown solid, MS: m/e = 592.1 (M+H⁺), using chemistry similar to that described in Reference compound 1, step 9 starting from ethyl (2RS)-2-[(6R)-6-fluoro-6,7-dihydro-5H-pyrrolo[1,2-c]imidazol-1-yl]-2-[6-iodo-1-oxo-4-(trifluoromethyl)isoindolin-2-yl]acetate *(Example 6, step 9)* and thiazol-2-amine.

### Step 11: (2RS)-2-[(6R)-6-Fluoro-6,7-dihydro-5H-pyrrolo[1,2-c]imidazol-1-yl]-2-[6-[2-[4-[[4-(hydroxymethyl)-1-piperidyl]methyl]phenyl]ethynyl]-1-oxo-4-(trifluoromethyl)isoindolin-2-yl]-N-thiazol-2-yl-acetamide

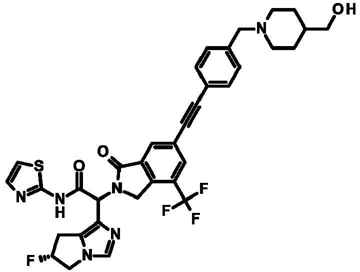

The title compound was obtained as white solid, MS: m/e = 693.4 (M+H⁺), using chemistry similar to that described in Reference compound 1, step 11 starting from (2RS)-2-[6-iodo-1-oxo-4-(trifluoromethyl)isoindolin-2-yl]-2-[(6R)-6-fluoro-6,7-dihydro-5H-pyrrolo[1,2-c]imidazol-1-yl]-N-thiazol-2-yl-acetamide *(Example 6, step 10)* and [1-[(4-ethynylphenyl)methyl]-4-piperidyl]methanol *(Example 3, step 1).*

### Example 7

### (2RS)-2-[4-(Difluoromethyl)-6-[4-(1-ethyl-4-piperidyl)phenyl]-1-oxo-isoindolin-2-yl]-2-[(6R)-6-fluoro-6,7-dihydro-5H-pyrrolo[1,2-c]imidazol-1-yl]-N-thiazol-2-yl-acetamide

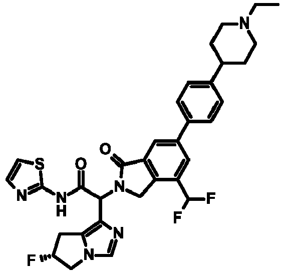

### Step 1: Ethyl (2RS)-2-[6-bromo-4-(difluoromethyl)-1-oxo-isoindolin-2-yl]-2-[(6R)-6-fluoro-6,7-dihydro-5H-pyrrolo[1,2-c]imidazol-1-yl]acetate

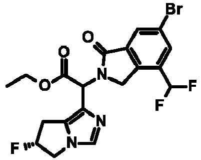

The title compound was obtained as an orange solid, MS: m/e = 473.9 (M+H⁺), using chemistry similar to that described in Reference compound 1, step 8 starting from ethyl (2RS)-2-amino-2-[(6R)-6-fluoro-6,7-dihydro-5H-pyrrolo[I,2-c]imidazol-I-yl]acetate *(Example 6, step 8)* and methyl 5-bromo-2-(bromomethyl)-3-(difluoromethyl)benzoate *(Example 4, step 3).*

### Step 2: (2RS)-2-[4-(Difluoromethyl)-6-[4-(1-ethyl-4-piperidyl)phenyl]-1-oxo-isoindolin-2-yl]-2-[(6R)-6-fluoro-6,7-dihydro-5H-pyrrolo[1,2-c]imidazol-1-yl]-N-thiazol-2-yl-acetamide

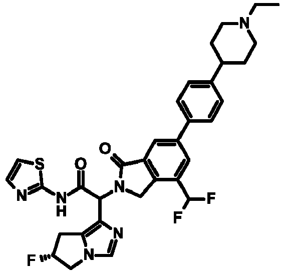

The title compound was obtained as a light brown solid, MS: m/e = 635.4 (M+H⁺), using chemistry similar to that described in Example 2, step 3 and Reference compound 1, step 9 starting from ethyl (2RS)-2-[6-bromo-4-(difluoromethyl)-1-oxo-isoindolin-2-yl]-2-[(6R)-6-fluoro-6,7-dihydro-5H-pyrrolo[1,2-c]imidazol-1-yl]acetate *(Example 7, step 1)* and 1-ethyl-4-[4-(4,4,5,5-tetramethyl-1,3,2-dioxaborolan-2-yl)phenyl]piperidine *(Example 2, step 2).*

### Example 8

### (2RS)-2-[4-(Difluoromethyl)-6-[4-[(3R,4R)-1-ethyl-3-fluoro-4-piperidyl]phenyl]-1-oxo-isoindolin-2-yl]-2-(6,7-dihydro-5H-pyrrolo[1,2-c]imidazol-1-yl)-N-thiazol-2-yl-acetamide and (2RS)-2-[4-(difluoromethyl)-6-[4-[(3S,4S)-1-ethyl-3-fluoro-4-piperidyl]phenyl]-1-oxo-isoindolin-2-yl]-2-(6,7-dihydro-5H-pyrrolo[1,2-c]imidazol-1-yl)-N-thiazol-2-yl-acetamide

### Step 1: tert-Butyl (3R,4R)-4-(4-bromophenyl)-3-hydroxy-piperidine-1-carboxylate and tert-butyl (3S,4S)-4-(4-bromophenyl)-3-hydroxy-piperidine-1-carboxylate

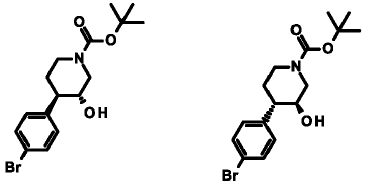

tert-Butyl 4-(4-bromophenyl)-3,6-dihydro-2H-pyridine-1-carboxylate *(*CAS 273727-44-9) (1.060 g, 3.13 mmol) was dissolved in 24 ml of THF and cooled to 0 °C. Borane tetrahydrofuran complex, 1.0 M solution in THF *(*CAS 14044-65-6*)* (6.6 ml, 6.6 mmol, 2.11 equiv.) was added dropwise at 0 °C. After the addition was complete, the ice bath was removed and the reaction mixture was stirred at room temperature for 16 hours. The reaction mixture was cooled to 0 °C. Sodium hydroxide (6 M in water) (1.7 ml, 10.2 mmol, 3.25 equiv.) was added and the reaction mixture was stirred at 0 °C for 10 minutes. Hydrogen peroxide, 35 wt.% solution in water (977 mg, 0.88 ml, 10.1 mmol, 3.21 equiv.) was added and the reaction mixture was stirred at 50 °C for 2 hours. The reaction mixture was cooled to room temperature and the excess of peroxide was quenched by addition of Na₂S₂O₃-solution (10 % in water). The mixture was extracted with ethyl acetate and saturated NaHCO₃-solution. The aqueous layer was extracted twice with ethyl acetate. The organic layers were combined, dried over sodium sulfate, filtered and concentrated on isolute^{®} to dryness. The crude product was purified by flash chromatography on a silica gel column eluting with an ethyl acetate:heptane 0:100 to 30:70 gradient. The desired products (894 mg, 76 % yield, purity = 95 %) were obtained as an off-white solid, MS: m/e = 300.0/302.0 (M-tBu+H⁺).

### Step 2: tert-Butyl (3R,4R)-4-(4-bromophenyl)-3-fluoro-piperidine-1-carboxylate and tert-butyl (3S,4S)-4-(4-bromophenyl)-3-fluoro-piperidine-1-carboxylate

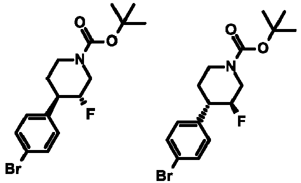

A mixture of tert-butyl (3R,4R)-4-(4-bromophenyl)-3-hydroxy-piperidine-1-carboxylate and tert-butyl (3S,4S)-4-(4-bromophenyl)-3-hydroxy-piperidine-1-carboxylate *(Example 8, step* 1) (0.492 g, 1.31 mmol, Eq: 1; purity=95%) was dissolved in 13 ml of dichlormethane and cooled to -76 °C. Bis(2-methoxyethyl)aminosulfur trifluoride *(*CAS 202289-38-1*)* (312 mg, 0.26 ml, 1.41 mmol, 1.07 equiv.) was added dropwise at -76 °C. The reaction mixture was allowed to slowly warm to room temperature and stirred for 16 hours. The reaction mixture was quenched with saturated NaHCOs-solution and extracted three times with Dichloromethane. The organic layers were combined, dried over sodium sulfate, filtered and concentrated on isolute^{®} to dryness. The crude product was purified by flash chromatography on a silica gel column eluting with an ethyl acetate:heptane 0:100 to 20:80 gradient. The desired products (300 mg, 61 % yield, purity = 95 %) were obtained as a colorless oil, MS: m/e = 302.0/304.0 (M-tBu+H⁺).

### Step 3: tert-Butyl (3R,4R)-3-fluoro-4-[4-(4,4,5,5-tetramethyl-1,3,2-dioxaborolan-2-yl)phenyl]piperidine-1-carboxylate and tert-butyl (3S,4S)-3-fluoro-4-r4-(4.4.5.5-tetramethyl-1,3.2-dioxaborolan-2-yl)phenyl]piperidine-1-carboxylate

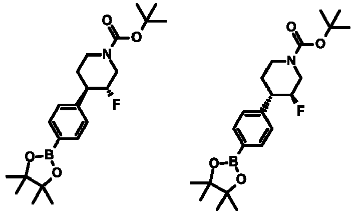

The title compounds were obtained as trans mixture and as an off-white foam, MS: m/e = 350.0 (M-tBu+H⁺), using chemistry similar to that described in Example 2, step 2 starting from tert-butyl (3R,4R)-4-(4-bromophenyl)-3-fluoro-piperidine-1-carboxylate and tert-butyl (3 S,4S)-4-(4-bromophenyl)-3-fluoro-piperidine-1-carboxylate *(Example 8, step 2).*

### Step 4: tert-Butyl (3R,4R)-4-[4-[7-(difluoromethyl)-2-[(1RS)-1-(6,7-dihydro-5H-pyrrolo[1,2-c]imidazol-1 -yl)-2-ethoxy-2-oxo-ethyl]-3 -oxo-isoindolin-5-yl]phenyl]-3-fluoro-piperidine-1 - carboxylate and tert-butyl (3S,4S)-4-[4-[7-(difluoromethyl)-2-[(1RS)-1-(6,7-dihydro-5H-pyrrolo[1,2-c]imidazol-1-yl)-2-ethoxy-2-oxo-ethyl]-3-oxo-isoindolin-5-yl]phenyl]-3-fluoro-piperidine-1 -carboxylate

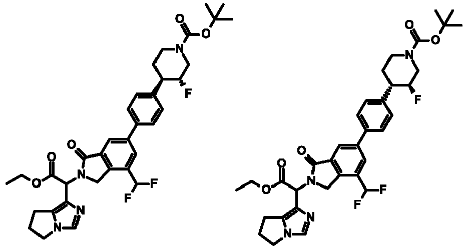

The title compounds were obtained as mixture and as a brown foam, MS: m/e = 653.4 (M+H⁺), using chemistry similar to that described in Example 2, step 3 starting from ethyl (2RS)-2-[6-bromo-4-(difluoromethyl)-1-oxo-isoindolin-2-yl]-2-(6,7-dihydro-5H-pyrrolo[1,2-c]imidazol-1-yl)acetate *(Example 4, step 4)* and tert-butyl (3R,4R)-3-fluoro-4-[4-(4,4,5,5-tetramethyl-1,3,2-dioxaborolan-2-yl)phenyl]piperidine-1-carboxylate and tert-butyl (3S,4S)-3-fluoro-4-[4-(4,4,5,5-tetramethyl-1,3,2-dioxaborolan-2-yl)phenyl]piperidine-1-carboxylate *(Example 8, step 3).*

### Step 5: tert-Butyl(3R,4R)-4-[4-[7-(difluoromethyl)-2-[(1RS)-1-(6,7-dihydro-5H-pyrrolo[1,2-c]imidazol-1-yl)-2-oxo-2-(thiazol-2-ylamino)ethyl]-3-oxo-isoindolin-5-yl]phenyl]-3-fluoro-piperidine-1-carboxylate and tert-butyl (3S,4S)-4-[4-[7-(difluoromethyl)-2-[(1RS)-1-(6,7-dihydro-5H-pyrrolo[1,2-c]imidazol-1-yl)-2-oxo-2-(thiazol-2-ylamino)ethyl]-3-oxo-isoindolin-5-yl]phenyl]-3-fluoro-piperidine-1-carboxylate

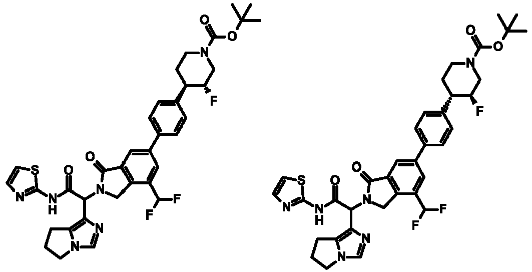

The title compounds were obtained as mixture and as a brown foam, MS: m/e = 707.4 (M+H⁺), using chemistry similar to that described in Reference compound 1, step 9 starting from a mixture of tert-butyl (3R,4R)-4-[4-[7-(difluoromethyl)-2-[(1RS)-1-(6,7-dihydro-5H-pyrrolo[1,2-c]imidazol-1-yl)-2-ethoxy-2-oxo-ethyl]-3-oxo-isoindolin-5-yl]phenyl]-3-fluoro-piperidine-1-carboxylate and tert-butyl (3S,4S)-4-[4-[7-(difluoromethyl)-2-[(1RS)-1-(6,7-dihydro-5H-pyrrolo[1,2-c]imidazol-1-yl)-2-ethoxy-2-oxo-ethyl]-3-oxo-isoindolin-5-yl]phenyl]-3-fluoro-piperidine-1-carboxylate *(Example 8, step 4)* and thiazol-2-amine.

### Step 6: (2RS)-2-14-(Difluoromethyl)-6-[4-[(3R,4R)-3-fluoro-4-piperidyl]phenyl]-1-oxo-isoindolin-2-yl]-2-(6,7-dihydro-5H-pyrrolo[1,2-c]imidazol-1-yl)-N-thiazol-2-yl-acetamide and (2RS)-2-[4-(difluoromethyl)-6-[4-(3S,4S)-3-fluoro-4-piperidyl]phenyl]-1-oxo-isoindolin-2-yl]-2-(6,7-dihydro-5H-pyrrolo[1,2-c]imidazol-1-yl)-N-thiazol-2-yl-acetamide

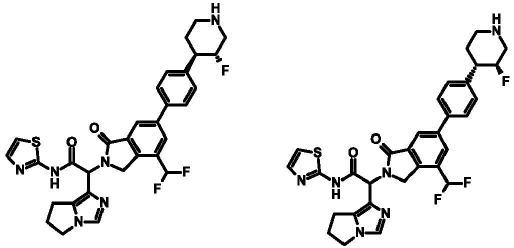

The title compounds were obtained as mixture and as a brown oil, MS: m/e = 607.3 (M+H⁺), using chemistry similar to that described in Example 6, step 3 starting from a mixture of tert-butyl (3R,4R)-4-[4-[7-(difluoromethyl)-2-[(1RS)-1-(6,7-dihydro-5H-pyrrolo[1,2-c]imidazol-1-yl)-2-oxo-2-(thiazol-2-ylamino)ethyl]-3-oxo-isoindolin-5-yl]phenyl]-3-fluoro-piperidine-1-carboxylate and tert-butyl (3S,4S)-4-[4-[7-(difluoromethyl)-2-[(1RS)-1-(6,7-dihydro-5H-pyrrolo[1,2-c]imidazol-1-yl)-2-oxo-2-(thiazol-2-ylamino)ethyl]-3-oxo-isoindolin-5-yl]phenyl]-3-fluoro-piperidine-1-carboxylate *(Example 8, step 5).*

### Step 7: (2RS)-2-[4-(Difluoromethyl)-6-[4-[(3R,4R)-1-ethyl-3-fluoro-4-piperidyl]phenyl]-1-oxo-isoindolin-2-yl]-2-(6,7-dihydro-5H-pyrrolo[1,2-c]imidazol-1-yl)-N-thiazol-2-yl-acetamide and (2RS)-2-[4-(difluoromethyl)-6-[4-[(3S,4S)-1-ethyl-3-fluoro-4-piperidyl]phenyl]-1-oxo-isoindolin-2-yl]-2-(6,7-dihydro-5H-pyrrolo[1,2-c]imidazol-1-yl)-N-thiazol-2-yl-acetamide

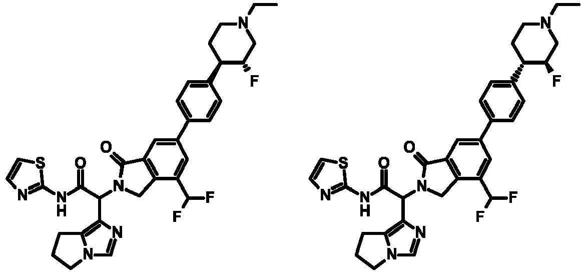

The title compounds were obtained as mixture and as an off-white foam, MS: m/e = 635.3 (M+H⁺), using chemistry similar to that described in Example 2, step 1 starting from a mixture of (2RS)-2-[4-(difluoromethyl)-6-[4-[(3R,4R)-3-fluoro-4-piperidyl]phenyl]-1-oxo-isoindolin-2-yl]-2-(6,7-dihydro-5H-pyrrolo[1,2-c]imidazol-1-yl)-N-thiazol-2-yl-acetamide and (2RS)-2-[4-(difluoromethyl)-6-[4-[(3S,4S)-3-fluoro-4-piperidyl]phenyl]-1-oxo-isoindolin-2-yl]-2-(6,7-dihydro-5H-pyrrolo[1,2-c]imidazol-1-yl)-N-thiazol-2-yl-acetamide *(Example 8, step 6)* and ethyl iodide.

### Example 9

### (2RS)-2-[(6R)-6-Fluoro-6,7-dihydro-5H-pyrrolo[1,2-c]imidazol-1-yl]-2-[6-[2-[4-[(6-hydroxy-2-azaspiro [3.3] heptan-2-yl)methyl] phenyl]ethynyl]-1-oxo-4-(trifluoromethyl)isoindolin-2-yl]-N-thiazol-2-yl-acetamide

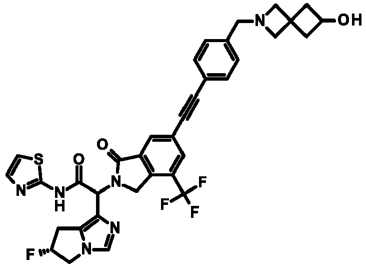

### Step 1: (2RS)-2-[(6R)-6-Fluoro-6,7-dihydro-5H-pyrrolo[1,2-c]imidazol-1-yl]-2-[6-[2-(4-formylphenyl)ethynyl]-1-oxo-4-(trifluoromethyl)isoindolin-2-yl]-N-thiazol-2-yl-acetamide

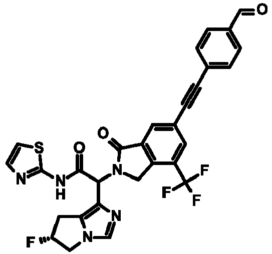

The title compound was obtained as light brown solid, MS: m/e = 594.2 (M+H⁺), using chemistry similar to that described in Reference compound 1, step 11 starting from (2RS)-2-[6-iodo-1-oxo-4-(trifluoromethyl)isoindolin-2-yl]-2-[(6R)-6-fluoro-6,7-dihydro-5H-pyrrolo[1,2-c]imidazol-1-yl]-N-thiazol-2-yl-acetamide *(Example 6, step 10)* and 4-ethynylbenzaldehyde.

### Step 2: (2RS)-2-[(6R)-6-Fluoro-6,7-dihydro-5H-pyrrolo[1,2-c]imidazol-1-yl]-2-[6-[2-[4-[(6-hydroxy-2-azaspiro[3.3]heptan-2-yl)methyl]phenyl]ethynyl]-1-oxo-4-(trifluoromethyl)isoindolin-2-yl]-N-thiazol-2-yl-acetamide

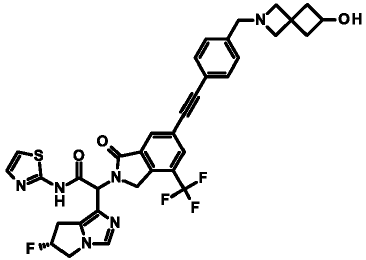

The title compound was obtained as a yellow solid, MS: m/e = 691.3 (M+H⁺), using chemistry similar to that described in Reference compound 1, step 10 starting from (2RS)-2-[(6R)-6-fluoro-6,7-dihydro-5H-pyrrolo[1,2-c]imidazol-1-yl]-2-[6-[2-(4-formylphenyl)ethynyl]-1-oxo-4-(trifluoromethyl)isoindolin-2-yl]-N-thiazol-2-yl-acetamide *(Example 9, step 1)* and 2-azaspiro[3.3]heptan-6-ol hydrochloride.

### Example 10

### (2RS)-2-[4-(Difluoromethyl)-6-[2-[4-[[4-(hydroxymethyl)-1-piperidyl]methyl]phenyl]ethynyl]-1-oxo-isoindolin-2-yl]-2-[(6R)-6-fluoro-6,7-dihydro-5H-pyrrolo[1,2-c] imidazol-1-yl]-N-thiazol-2-yl-acetamide

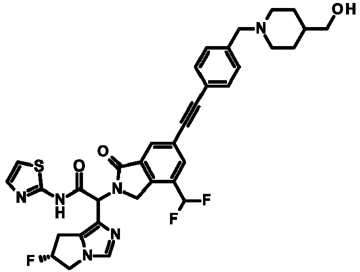

### Step 1: (2RS)-2-[6-Bromo-4-(difluoromethyl)-1-oxo-isoindolin-2-yl]-2-[(6R)-6-fluoro-6,7-dihydro-5H-pyrrolo[1,2-c]imidazol-1-yl]-N-thiazol-2-yl-acetamide

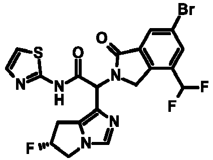

The title compound was obtained as a light brown solid, MS: m/e = 528.1 (M+H⁺), using chemistry similar to that described in Reference compound 1, step 9 starting from ethyl (2RS)-2-[6-bromo-4-(difluoromethyl)-1-oxo-isoindolin-2-yl]-2-[(6R)-6-fluoro-6,7-dihydro-5H-pyrrolo[1,2-c]imidazol-1-yl]acetate *(Example 7, step 1)* and thiazol-2-amine.

### Step 2: (2RS)-2-[4-(Difluoromethyl)-6-[2-[4-[[4-(hydroxymethyl)-1-piperidyl]methyl]phenyl]ethynyl]-1-oxo-isoindolin-2-yl]-2-[(6R)-6-fluoro-6,7-dihydro-5H-pyrrolo[1,2-climidazol-1-yl]-N-thiazol-2-yl-acetamide

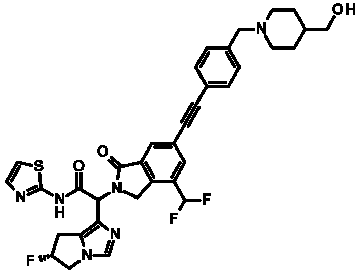

The title compound was obtained as light brown solid, MS: m/e = 675.4 (M+H⁺), using chemistry similar to that described in Reference compound 1, step 11 starting from (2RS)-2-[6-bromo-4-(difluoromethyl)-1-oxo-isoindolin-2-yl]-2-[(6R)-6-fluoro-6,7-dihydro-5H-pyrrolo[1,2-c]imidazol-1-yl]-N-thiazol-2-yl-acetamide *(Example 10, step 1)* and [1-[(4-ethynylphenyl)methyl]-4-piperidyl]methanol *(Example 3, step 1).*

### Example 11

### (2RS)-2-[(6R)-6-fluoro-6,7-dihydro-5H-pyrrolo[1,2-c]imidazol-1-yl]-2-[6-[2-[4-[[(4RS)-4-hydroxyazepan-1-yl]methyl]phenyl]ethynyl]-1-oxo-4-(trifluoromethyl)isoindolin-2-yl]-N-thiazol-2-yl-acetamide

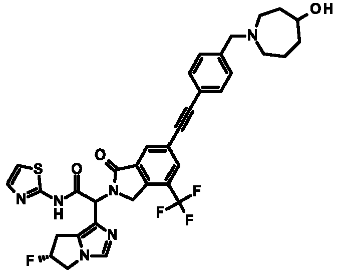

The title compound was obtained as a white solid, MS: m/e = 693.3 (M+H⁺), using chemistry similar to that described in Reference compound 1, step 10 starting from (2RS)-2-[(6R)-6-fluoro-6,7-dihydro-5H-pyrrolo[1,2-c]imidazol-1-yl]-2-[6-[2-(4-formylphenyl)ethynyl]-1-oxo-4-(trifluoromethyl)isoindolin-2-yl]-N-thiazol-2-yl-acetamide *(Example 9, step 1)* and azepan-4-ol.

## Claims

1. A compound selected from
2-(6,7-dihydro-5H-pyrrolo[1,2-c]imidazol-1-yl)-2-[6-[4-(1-ethyl-4-piperidyl)phenyl]-1-oxo-4-(trifluoromethyl)isoindolin-2-yl]-N-thiazol-2-yl-acetamide;
2-(6,7-dihydro-5H-pyrrolo[1,2-c]imidazol-1-yl)-2-[6-[2-[4-[[4-(hydroxymethyl)-1-piperidyl]methyl]phenyl]ethynyl]-1-oxo-4-(trifluoromethyl)isoindolin-2-yl]-N-thiazol-2-yl-acetamide;
2-[4-(difluoromethyl)-6-[4-(1-ethyl-4-piperidyl)phenyl]-1-oxo-isoindolin-2-yl]-2-(6,7-dihydro-5H-pyrrolo[1,2-c]imidazol-1 -yl)-N-thiazol-2-yl-acetamide;
2-[4-(difluoromethyl)-6-[2-[4-[[4-(hydroxymethyl)-1-piperidyl]methyl]phenyl]ethynyl]-1-oxo-isoindolin-2-yl]-2-(6,7-dihydro-5H-pyrrolo[1,2-c]imidazol-1-yl)-N-thiazol-2-yl-acetamide;
2-[6-[2-[4-[[4-(hydroxymethyl)-1-piperidyl]methyl]phenyl]ethynyl]-1-oxo-4-(trifluoromethyl)isoindolin-2-yl]-2-[rac-(6R)-6-fluoro-6,7-dihydro-5H-pyrrolo[1,2-c]imidazol-1-yl]-N-thiazol-2-yl-acetamide;
2-[4-(difluoromethyl)-6-[4-(1-ethyl-4-piperidyl)phenyl]-1-oxo-isoindolin-2-yl]-2-[rac-(6R)-6-fluoro-6,7-dihydro-5H-pyrrolo[1,2-c]imidazol-1-yl]-N-thiazol-2-yl-acetamide;
2-[4-(difluoromethyl)-1-oxo-6-[4-[rac-(3R,4R)-1-ethyl-3-fluoro-4-piperidyl]phenyl]isoindolin-2-yl]-2-(6,7-dihydro-5H-pyrrolo[1,2-c]imidazol-1-yl)-N-thiazol-2-yl-acetamide;
2-[6-[2-[4-[(6-hydroxy-2-azaspiro[3.3]heptan-2-yl)methyl]phenyl]ethynyl]-1-oxo-4-(trifluoromethyl)isoindolin-2-yl]-2-[rac-(6R)-6-fluoro-6,7-dihydro-5H-pyrrolo[1,2-c]imidazol-1-yl]-N-thiazol-2-yl-acetamide;
2-[4-(difluoromethyl)-6-[2-[4-[[4-(hydroxymethyl)-1-piperidyl]methyl]phenyl]ethynyl]-1-oxo-isoindolin-2-yl]-2-[rac-(6R)-6-fluoro-6,7-dihydro-5H-pyrrolo[1,2-c]imidazol-1-yl]-N-thiazol-2-yl-acetamide;
2-[6-[2-[4-[(4-hydroxyazepan-1-yl)methyl]phenyl]ethynyl]-1-oxo-4-(trifluoromethyl)isoindolin-2-yl]-2-[rac-(6R)-6-fluoro-6,7-dihydro-5H-pyrrolo[1,2-c]imidazol-1-yl]-N-thiazol-2-yl-acetamide;
or pharmaceutically acceptable salts.

2. A compound according to claim 1 selected from
2-(6,7-dihydro-5H-pyrrolo[1,2-c]imidazol-1-yl)-2-[6-[4-(1 -ethyl-4-piperidyl)phenyl]-1-oxo-4-(trifluoromethyl)isoindolin-2-yl]-N-thiazol-2-yl-acetamide;
2-(6,7-dihydro-5H-pyrrolo[1,2-c]imidazol-1-yl)-2-[6-[2-[4-[[4-(hydroxymethyl)-1-piperidyl]methyl]phenyl]ethynyl]-1-oxo-4-(trifluoromethyl)isoindolin-2-yl]-N-thiazol-2-yl-acetamide;
2-[4-(difluoromethyl)-6-[4-(1-ethyl-4-piperidyl)phenyl]-1-oxo-isoindolin-2-yl]-2-(6,7-dihydro-5H-pyrrolo[1,2-c]imidazol-1-yl)-N-thiazol-2-yl-acetamide;
2-[4-(difluoromethyl)-6-[2-[4-[[4-(hydroxym ethyl)-1-piperidyl]methyl]phenyl]ethynyl]-1-oxo-isoindolin-2-yl]-2-(6,7-dihydro-5H-pyrrolo[1,2-c]imidazol-1-yl)-N-thiazol-2-yl-acetamide;
2-[6-[2-[4-[[4-(hydroxymethyl)-1-piperidyl]methyl]phenyl]ethynyl]-1-oxo-4-(trifluoromethyl)isoindolin-2-yl]-2-[rac-(6R)-6-fluoro-6,7-dihydro-5H-pyrrolo[1,2-c]imidazol-1-yl]-N-thiazol-2-yl-acetamide;
2-[4-(difluoromethyl)-6-[4-(1-ethyl-4-piperidyl)phenyl]-1-oxo-isoindolin-2-yl]-2-[rac-(6R)-6-fluoro-6,7-dihydro-5H-pyrrolo[1,2-c]imidazol-1-yl]-N-thiazol-2-yl-acetamide;
2-[4-(difluoromethyl)-1-oxo-6-[4-[rac-(3R,4R)-1-ethyl-3-fluoro-4-piperidyl]phenyl]isoindolin-2-yl]-2-(6,7-dihydro-5H-pyrrolo[1,2-c]imidazol-1-yl)-N-thiazol-2-yl-acetamide;
2-[6-[2-[4-[(6-hydroxy-2-azaspiro[3.3]heptan-2-yl)methyl]phenyl]ethynyl]-1-oxo-4-(trifluoromethyl)isoindolin-2-yl]-2-[rac-(6R)-6-fluoro-6,7-dihydro-5H-pyrrolo[1,2-c]imidazol-1-yl]-N-thiazol-2-yl-acetamide;
2-[4-(difluoromethyl)-6-[2-[4-[[4-(hydroxymethyl)-1-piperidyl]methyl]phenyl]ethynyl]-1-oxo-isoindolin-2-yl]-2-[rac-(6R)-6-fluoro-6,7-dihydro-5H-pyrrolo[1,2-c]imidazol-1-yl]-N-thiazol-2-yl-acetamide;
2-[6-[2-[4-[(4-hydroxyazepan-1-yl)methyl]phenyl]ethynyl]-1-oxo-4-(trifluoromethyl)isoindolin-2-yl]-2-[rac-(6R)-6-fluoro-6,7-dihydro-5H-pyrrolo[1,2-c]imidazol-1-yl]-N-thiazol-2-yl-acetamide.

3. A compound according to any one of claims 1 or 2 for use as therapeutically active substance.

4. A pharmaceutical composition comprising a compound according to any one of claims 1 or 2 and a therapeutically inert carrier.

5. A compound according to any one of claims 1 or 2 for use in the treatment or prophylaxis of cancer.

6. A compound according to any one of claims 1 to 2 for use in the treatment or prophylaxis of non-small cell lung cancer.

## Patentansprüche

1. Verbindung, ausgewählt aus
2-(6,7-Dihydro-5H-pyrrolo[1,2-c]imidazol-1-yl)-2-[6-[4-(1-ethyl-4-piperidyl)phenyl]-1-oxo-4-(trifluormethyl)isoindolin-2-yl]-N-thiazol-2-yl-acetamid;
2-(6,7-Dihydro-5H-pyrrolo[1,2-c]imidazol-1-yl)-2-[6-[2-[4-[[4-(hydroxymethyl)-1-piperidyl]methyl]phenyl]ethinyl]-1-oxo-4-(trifluormethyl)isoindolin-2-yl]-N-thiazol-2-yl-acetamid;
2-[4-(Difluormethyl)-6-[4-(1-ethyl-4-piperidyl)phenyl]-1-oxo-isoindolin-2-yl]-2-(6,7-dihydro-5H-pyrrolo[1,2-c]imidazol-1-yl)-N-thiazol-2-yl-acetamid;
2-[4-(Difluormethyl)-6-[2-[4-[[4-(hydroxymethyl)-1-piperidyl]methyl]phenyl]ethinyl]-1-oxo-isoindolin-2-yl]-2-(6,7-dihydro-5H-pyrrolo[1,2-c]imidazol-1-yl)-N-thiazol-2-yl-acetamid;
2-[6-[2-[4-[[4-(Hydroxymethyl)-1-piperidyl]methyl]phenyl]ethinyl]-1-oxo-4-(trifluormethyl)isoindolin-2-yl]-2-[rac-(6R)-6-fluor-6,7-dihydro-5H-pyrrolo[1,2-c]imidazol-1-yl]-N-thiazol-2-yl-acetamid;
2-[4-(Difluormethyl)-6-[4-(1-ethyl-4-piperidyl)phenyl]-1-oxo-isoindolin-2-yl]-2-[rac-(6R)-6-fluor-6,7-dihydro-5H-pyrrolo[1,2-c]imidazol-1-yl]-N-thiazol-2-yl-acetamid;
2-[4-(Difluormethyl)-1-oxo-6-[4-[rac-(3R,4R)-1-ethyl-3-fluor-4-piperidyl]phenyl]isoindolin-2-yl]-2-(6,7-dihydro-5H-pyrrolo[1,2-c]imidazol-1-yl)-N-thiazol-2-yl-acetamid;
2-[6-[2-[4-[(6-Hydroxy-2-azaspiro[3.3]heptan-2-yl)methyl]phenyl]ethinyl]-1-oxo-4-(trifluormethyl)isoindolin-2-yl]-2-[rac-(6R)-6-fluor-6,7-dihydro-5H-pyrrolo[1,2-c]imidazol-1-yl]-N-thiazol-2-yl-acetamid;
2-[4-(Difluormethyl)-6-[2-[4-[[4-(hydroxymethyl)-1-piperidyl]methyl]phenyl]ethinyl]-1-oxo-isoindolin-2-yl]-2-[rac-(6R)-6-fluor-6,7-dihydro-5H-pyrrolo[1,2-c]imidazol-1-yl]-N-thiazol-2-yl-acetamid;
2-[6-[2-[4-[(4-Hydroxyazepan-1-yl)methyl]phenyl]ethinyl]-1-oxo-4-(trifluormethyl)isoindolin-2-yl]-2-[rac-(6R)-6-fluor-6,7-dihydro-5H-pyrrolo[1,2-c]imidazol-1-yl]-N-thiazol-2-yl-acetamid;
oder pharmazeutisch unbedenklichen Salzen.

2. Verbindung nach Anspruch 1, ausgewählt aus
2-(6,7-Dihydro-5H-pyrrolo[1,2-c]imidazol-1-yl)-2-[6-[4-(1-ethyl-4-piperidyl)phenyl]-1-oxo-4-(trifluormethyl)isoindolin-2-yl]-N-thiazol-2-yl-acetamid;
2-(6,7-Dihydro-5H-pyrrolo[1,2-c]imidazol-1-yl)-2-[6-[2-[4-[[4-(hydroxymethyl)-1-piperidyl]methyl]phenyl]ethinyl]-1-oxo-4-(trifluormethyl)isoindolin-2-yl]-N-thiazol-2-yl-acetamid;
2-[4-(Difluormethyl)-6-[4-(1-ethyl-4-piperidyl)phenyl]-1-oxo-isoindolin-2-yl]-2-(6,7-dihydro-5H-pyrrolo[1,2-c]imidazol-1-yl)-N-thiazol-2-yl-acetamid;
2-[4-(Difluormethyl)-6-[2-[4-[[4-(hydroxymethyl)-1-piperidyl]methyl]phenyl]ethinyl]-1-oxo-isoindolin-2-yl]-2-(6,7-dihydro-5H-pyrrolo[1,2-c]imidazol-1-yl)-N-thiazol-2-yl-acetamid;
2-[6-[2-[4-[[4-(Hydroxymethyl)-1-piperidyl]methyl]phenyl]ethinyl]-1-oxo-4-(trifluormethyl)isoindolin-2-yl]-2-[rac-(6R)-6-fluor-6,7-dihydro-5H-pyrrolo[1,2-c]imidazol-1-yl]-N-thiazol-2-yl-acetamid;
2-[4-(Difluormethyl)-6-[4-(1-ethyl-4-piperidyl)phenyl]-1-oxo-isoindolin-2-yl]-2-[rac-(6R)-6-fluor-6,7-dihydro-5H-pyrrolo[1,2-c]imidazol-1-yl]-N-thiazol-2-yl-acetamid;
2-[4-(Difluormethyl)-1-oxo-6-[4-[rac-(3R,4R)-1-ethyl-3-fluor-4-piperidyl]phenyl]isoindolin-2-yl]-2-(6,7-dihydro-5H-pyrrolo[1,2-c]imidazol-1-yl)-N-thiazol-2-yl-acetamid;
2-[6-[2-[4-[(6-Hydroxy-2-azaspiro[3.3]heptan-2-yl)methyl]phenyl]ethinyl]-1-oxo-4-(trifluormethyl)isoindolin-2-yl]-2-[rac-(6R)-6-fluor-6,7-dihydro-5H-pyrrolo[1,2-c]imidazol-1-yl]-N-thiazol-2-yl-acetamid;
2-[4-(Difluormethyl)-6-[2-[4-[[4-(hydroxymethyl)-1-piperidyl]methyl]phenyl]ethinyl]-1-oxo-isoindolin-2-yl]-2-[rac-(6R)-6-fluor-6,7-dihydro-5H-pyrrolo[1,2-c]imidazol-1-yl]-N-thiazol-2-yl-acetamid;
2-[6-[2-[4-[(4-Hydroxyazepan-1-yl)methyl]phenyl]ethinyl]-1-oxo-4-(trifluormethyl)isoindolin-2-yl]-2-[rac-(6R)-6-fluor-6,7-dihydro-5H-pyrrolo[1,2-c]imidazol-1-yl]-N-thiazol-2-yl-acetamid.

3. Verbindung nach einem der Ansprüche 1 oder 2 zur Verwendung als therapeutisch wirksame Substanz.

4. Pharmazeutische Zusammensetzung, umfassend eine Verbindung nach einem der Ansprüche 1 oder 2 und einen therapeutisch inerten Träger.

5. Verbindung nach einem der Ansprüche 1 oder 2 zur Verwendung bei der Behandlung oder Prophylaxe von Krebs.

6. Verbindung nach einem der Ansprüche 1 bis 2 zur Verwendung bei der Behandlung oder Prophylaxe von nicht-kleinzelligem Lungenkrebs.

## Revendications

1. Composé choisi parmi
le 2-(6,7-dihydro-5H-pyrrolo[1,2-c]imidazol-1-yl)-2-[6-[4-(1-éthyl-4-pipéridyl)phényl]-1-oxo-4-(trifluorométhyl)isoindolin-2-yl]-N-thiazol-2-yl-acétamide ;
le 2-(6,7-dihydro-5H-pyrrolo[1,2-c]imidazol-1-yl)-2-[6-[2-[4-[[4-(hydroxyméthyl)-1-pipéridyl]méthyl]phényl]éthynyl]-1-oxo-4-(trifluorométhyl)isoindolin-2-yl]-N-thiazol-2-yl-acétamide ;
le 2-[4-(difluorométhyl)-6-[4-(1-éthyl-4-pipéridyl)phényl]-1-oxo-isoindolin-2-yl]-2-(6,7-dihydro-5H-pyrrolo[1,2-c]imidazol-1-yl)-N-thiazol-2-yl-acétamide ;
le 2-[4-(difluorométhyl)-6-[2-[4-[[4-(hydroxyméthyl)-1-pipéridyl]méthyl]phényl]éthynyl]-1-oxo-isoindolin-2-yl]-2-(6,7-dihydro-5H-pyrrolo[1,2-c]imidazol-1-yl)-N-thiazol-2-yl-acétamide ;
le 2-[6-[2-[4-[[4-(hydroxyméthyl)-1-pipéridyl]méthyl]phényl]éthynyl]-1-oxo-4-(trifluorométhyl)isoindolin-2-yl]-2-[rac-(6R)-6-fluoro-6,7-dihydro-5H-pyrrolo[1,2-c]imidazol-1-yl]-N-thiazol-2-yl-acétamide ;
le 2-[4-(difluorométhyl)-6-[4-(1-éthyl-4-pipéridyl)phényl]-1-oxo-isoindolin-2-yl]-2-[rac-(6R)-6-fluoro-6,7-dihydro-5H-pyrrolo[1,2-c]imidazol-1-yl]-N-thiazol-2-yl-acétamide ;
le 2-[4-(difluorométhyl)-1-oxo-6-[4-[rac-(3R,4R)-1-éthyl-3-fluoro-4-pipéridyl]phényl]isoindolin-2-yl]-2-(6,7-dihydro-5H-pyrrolo[1,2-c]imidazol-1-yl)-N-thiazol-2-yl-acétamide ;
le 2-[6-[2-[4-[(6-hydroxy-2-azaspiro[3.3]heptan-2-yl)méthyl]phényl]éthynyl]-1-oxo-4-(trifluorométhyl)isoindolin-2-yl]-2-[rac-(6R)-6-fluoro-6,7-dihydro-5H-pyrrolo[1,2-c]imidazol-1-yl]-N-thiazol-2-yl-acétamide ;
le 2-[4-(difluorométhyl)-6-[2-[4-[[4-(hydroxyméthyl)-1-pipéridyl]méthyl]phényl]éthynyl]-1-oxo-isoindolin-2-yl]-2-[rac-(6R)-6-fluoro-6,7-dihydro-5H-pyrrolo[1,2-c]imidazol-1-yl]-N-thiazol-2-yl-acétamide ;
le 2-[6-[2-[4-[(4-hydroxyazépan-1-yl)méthyl]phényl]éthynyl]-1-oxo-4-(trifluorométhyl)isoindolin-2-yl]-2-[rac-(6R)-6-fluoro-6,7-dihydro-5H-pyrrolo[1,2-c]imidazol-1-yl]-N-thiazol-2-yl-acétamide ;
ou des sels pharmaceutiquement acceptables.

2. Composé selon la revendication 1 choisi parmi
le 2-(6,7-dihydro-5H-pyrrolo[1,2-c]imidazol-1-yl)-2-[6-[4-(1-éthyl-4-pipéridyl)phényl]-1-oxo-4-(trifluorométhyl)isoindolin-2-yl]-N-thiazol-2-yl-acétamide ;
le 2-(6,7-dihydro-5H-pyrrolo[1,2-c]imidazol-1-yl)-2-[6-[2-[4-[[4-(hydroxyméthyl)-1-pipéridyl]méthyl]phényl]éthynyl]-1-oxo-4-(trifluorométhyl)isoindolin-2-yl]-N-thiazol-2-yl-acétamide ;
le 2-[4-(difluorométhyl)-6-[4-(1-éthyl-4-pipéridyl)phényl]-1-oxo-isoindolin-2-yl]-2-(6,7-dihydro-5H-pyrrolo[1,2-c]imidazol-1-yl)-N-thiazol-2-yl-acétamide ;
le 2-[4-(difluorométhyl)-6-[2-[4-[[4-(hydroxyméthyl)-1-pipéridyl]méthyl]phényl]éthynyl]-1-oxo-isoindolin-2-yl]-2-(6,7-dihydro-5H-pyrrolo[1,2-c]imidazol-1-yl)-N-thiazol-2-yl-acétamide ;
le 2-[6-[2-[4-[[4-(hydroxyméthyl)-1-pipéridyl]méthyl]phényl]éthynyl]-1-oxo-4-(trifluorométhyl)isoindolin-2-yl]-2-[rac-(6R)-6-fluoro-6,7-dihydro-5H-pyrrolo[1,2-c]imidazol-1-yl]-N-thiazol-2-yl-acétamide ;
le 2-[4-(difluorométhyl)-6-[4-(1-éthyl-4-pipéridyl)phényl]-1-oxo-isoindolin-2-yl]-2-[rac-(6R)-6-fluoro-6,7-dihydro-5H-pyrrolo[1,2-c]imidazol-1-yl]-N-thiazol-2-yl-acétamide ;
le 2-[4-(difluorométhyl)-1-oxo-6-[4-[rac-(3R,4R)-1-éthyl-3-fluoro-4-pipéridyl]phényl]isoindolin-2-yl]-2-(6,7-dihydro-5H-pyrrolo[1,2-c]imidazol-1-yl)-N-thiazol-2-yl-acétamide ;
le 2-[6-[2-[4-[(6-hydroxy-2-azaspiro[3.3]heptan-2-yl)méthyl]phényl]éthynyl]-1-oxo-4-(trifluorométhyl)isoindolin-2-yl]-2-[rac-(6R)-6-fluoro-6,7-dihydro-5H-pyrrolo[1,2-c]imidazol-1-yl]-N-thiazol-2-yl-acétamide ;
le 2-[4-(difluorométhyl)-6-[2-[4-[[4-(hydroxyméthyl)-1-pipéridyl]méthyl]phényl]éthynyl]-1-oxo-isoindolin-2-yl]-2-[rac-(6R)-6-fluoro-6,7-dihydro-5H-pyrrolo[1,2-c]imidazol-1-yl]-N-thiazol-2-yl-acétamide ;
le 2-[6-[2-[4-[(4-hydroxyazépan-1-yl)méthyl]phényl]éthynyl]-1-oxo-4-(trifluorométhyl)isoindolin-2-yl]-2-[rac-(6R)-6-fluoro-6,7-dihydro-5H-pyrrolo[1,2-c]imidazol-1-yl]-N-thiazol-2-yl-acétamide.

3. Composé selon l'une quelconque des revendications 1 ou 2 pour une utilisation comme substance thérapeutiquement active.

4. Composition pharmaceutique comprenant un composé selon l'une quelconque des revendications 1 ou 2 et un véhicule thérapeutiquement inerte.

5. Composé selon l'une quelconque des revendications 1 ou 2 pour une utilisation dans le traitement ou la prophylaxie du cancer.

6. Composé selon l'une quelconque des revendications 1 à 2 pour une utilisation dans le traitement ou la prophylaxie du cancer du poumon non à petites cellules.
